# DEMANDE DE BREVET EUROPEEN

(11) **EP 4 647 036 A1**
(43) Date de publication de la demande: **12.11.2025**
(21) Numéro de dépôt: 25203287.5
(22) Date de dépôt: 19.07.2018
(51) Int. Cl.: A61C 7/00, G06V 10/764, G06V 10/82, G06T 7/62

(54) **PROCEDE D'ANALYSE D'UNE IMAGE D'UNE ARCADE DENTAIRE**

(30) Priorité: 21.07.2017 FR 1756953
(62) Demande divisionnaire de: 21169818.8
(71) Demandeur: Dental Monitoring, 75017 Paris (FR)
(72) Inventeur: SALAH, Philippe, 75017 PARIS (FR); PELLISSARD, Thomas, 75004 PARIS (FR); GHYSELINK, Guillaume, 59169 CANTIN (FR); DEBRAUX, Laurent, 75020 PARIS (FR)
(74) Mandataire: Ipsilon

(57) **Abrégé**

Procédé d'analyse d'une image d'une arcade dentaire d'un patient, dite « image d'analyse », comportant l'acquisition avec un téléphone mobile et par le patient de l'image d'analyse, l'image d'analyse étant en couleurs et choisie parmi une photographie et une image extraite d'un film, le téléphone mobile étant écarté de l'arcade dentaire de plus de 5 cm lors de l'acquisition, ledit procédé d'analyse comportant les étapes suivantes :
1') création d'une base d'apprentissage comportant plus de 1 000 images d'arcades dentaires, ou « images historiques », chaque image historique comportant une valeur d'attribut pour un attribut d'image, ou « valeur d'attribut d'image » ;
2') entraînement d'au moins un réseau de neurones, au moyen de la base d'apprentissage, en présentant, en entrée du réseau de neurones, les images historiques, afin que le réseau de neurones apprenne progressivement à reconnaître sur une image, des motifs, et à les associer à des valeurs d'attribut d'image ;
3') soumission de l'image d'analyse au réseau de neurones de manière qu'il détermine, pour ladite image d'analyse, au moins une probabilité relative à ladite valeur d'attribut d'image, et détermination, en fonction de ladite probabilité, d'une valeur pour ledit attribut d'image pour l'image d'analyse ;
ledit attribut d'image étant relatif à
- une position et/ou une orientation et/ou une calibration de l'appareil d'acquisition utilisé pour acquérir ladite image d'analyse, ou
- une qualité de l'image d'analyse, ou
- la luminosité, au contraste ou à la netteté de l'image d'analyse,

## Description

### Domaine technique

La présente invention concerne le domaine de l'analyse d'images d'arcades dentaires.

### Etat de la technique

Les traitements orthodontiques les plus récents utilisent des images pour évaluer les situations thérapeutiques. Cette évaluation est classiquement effectuée par un orthodontiste, ce qui nécessite que le patient transmette ces images à l'orthodontiste, voire qu'il prenne un rendez-vous.

Il existe un besoin permanent pour un procédé facilitant l'analyse des images d'arcades dentaires de patients.

Un but de l'invention est de répondre à ce besoin.

### Résumé de l'invention

L'invention propose un procédé d'analyse d'une image, dite « image d'analyse », d'une arcade dentaire d'un patient, procédé dans lequel l'image d'analyse est soumise à un dispositif d'apprentissage profond, de préférence un réseau de neurones, afin de déterminer au moins une valeur d'un attribut de dent relative à une dent représentée sur l'image d'analyse, et/ou au moins une valeur d'un attribut d'image relative à l'image d'analyse.

### Analyse par dent

L'invention propose en particulier un procédé d'analyse détaillée d'une image dite « image d'analyse » d'une arcade dentaire d'un patient, ledit procédé comportant les étapes suivantes :
1) création d'une base d'apprentissage comportant plus de 1 000 images d'arcades dentaires, ou « images historiques », chaque image historique comportant une ou plusieurs zones représentant chacune une dent, ou « zones de dent historiques », à chacune desquelles, pour au moins un attribut de dent, une valeur d'attribut de dent est affectée ;
2) entraînement d'au moins un dispositif d'apprentissage profond, de préférence un réseau de neurones, au moyen de la base d'apprentissage ;
3) soumission de l'image d'analyse audit au moins un dispositif d'apprentissage profond de manière qu'il détermine au moins une probabilité relative à une valeur d'attribut d'au moins une dent représentée sur une zone représentant, au moins partiellement, ladite dent dans l'image d'analyse, ou « zone de dent d'analyse » ;
4) détermination, en fonction de ladite probabilité, de la présence d'une dent de ladite arcade à une position représentée par ladite zone de dent d'analyse, et de la valeur d'attribut de ladite dent.

Un premier dispositif d'apprentissage profond, de préférence un réseau de neurones, peut être en particulier mis en œuvre pour évaluer une probabilité relative à la présence, à un emplacement de ladite image d'analyse, d'une zone de dent d'analyse.

Un deuxième dispositif d'apprentissage profond, de préférence un réseau de neurones, peut être en particulier mis en œuvre pour évaluer une probabilité relative au type de dent représenté dans une zone de dent d'analyse.

Comme on le verra plus en détail dans la suite de la description, un procédé d'analyse détaillée selon l'invention permet avantageusement de reconnaître immédiatement le contenu de l'image d'analyse.

L'image d'analyse peut être avantageusement classée automatiquement. Elle est par ailleurs immédiatement exploitable par un programme informatique.

L'invention repose sur l'utilisation d'un dispositif d'apprentissage profond, de préférence un réseau de neurones, dont la performance est directement liée à la richesse de la base d'apprentissage. Il existe donc également un besoin pour un procédé permettant d'enrichir rapidement la base d'apprentissage.

L'invention concerne donc encore un procédé d'enrichissement d'une base d'apprentissage, notamment destinée à la mise en œuvre d'un procédé d'analyse détaillée selon l'invention, ledit procédé d'enrichissement comportant les étapes suivantes :
A) à un instant « actualisé », réalisation d'un modèle d'une arcade dentaire d'un patient, ou « modèle de référence actualisé », et segmentation du modèle de référence actualisé de manière à réaliser, pour chaque dent, un « modèle de dent », et pour au moins un attribut de dent, affectation d'une valeur d'attribut de dent à chaque modèle de dent ;
B) de préférence moins de 6 mois, de préférence moins de 2 mois, de préférence moins de 1 mois, de préférence moins de 15 jours, de préférence moins de 1 semaine, de préférence moins de 1 jour avant ou après l'instant actualisé, de préférence sensiblement à l'instant actualisé, acquisition d'au moins une, de préférence d'au moins trois, de préférence d'au moins dix, de préférence d'au moins cent images de ladite arcade, ou « images actualisées », dans des conditions d'acquisition réelles respectives ;
C) pour chaque image actualisée, recherche de conditions d'acquisition virtuelles adaptées pour une acquisition d'une image du modèle de référence actualisé, dite « image de référence », présentant une concordance maximale avec l'image actualisée dans lesdites conditions d'acquisition virtuelles, et acquisition de ladite image de référence ;
D) identification, dans l'image de référence, d'au moins une zone représentant un modèle de dent, ou « zone de dent de référence » et, par comparaison de l'image actualisée et de l'image de référence, détermination, dans l'image actualisée, d'une zone représentant ledit modèle de dent, ou « zone de dent actualisée » ;
E) affectation, à ladite zone de dent actualisée, de la ou des valeurs d'attribut de dent dudit modèle de dent ;
F) ajout de l'image actualisée enrichie d'un descriptif de ladite zone de dent actualisée et de sa ou ses valeurs d'attribut de dent, ou « image historique », dans la base d'apprentissage.

En particulier, chaque exécution du procédé décrit dans WO 2016/066651 génère de préférence plus de trois, plus de dix, de préférence plus de cent images actualisées qui, par un traitement automatisé au moyen du modèle de référence actualisé, peuvent produire autant d'images historiques.

Dans un mode de réalisation particulier, le procédé d'enrichissement d'une base d'apprentissage comporte, à la place des étapes A) à C), les étapes suivantes :
A') à un instant initial, réalisation d'un modèle d'une arcade dentaire d'un patient, ou « modèle de référence initial », et segmentation du modèle de référence initial de manière à réaliser, pour chaque dent, un « modèle de dent », et pour au moins un attribut de dent, affectation d'une valeur d'attribut de dent à chaque modèle de dent ;
B') à un instant actualisé, par exemple espacé de plus de quinze jours, de préférence de plus d'un mois, voire de plus de deux mois de l'instant initial, acquisition d'au moins une, de préférence d'au moins trois, de préférence d'au moins dix, de préférence d'au moins cent images de ladite arcade, ou « images actualisées », dans des conditions d'acquisition réelles respectives ;
C') pour chaque image actualisée, recherche, par déformation du modèle de référence initial, d'un modèle de référence actualisé et de conditions d'acquisition virtuelles adaptés pour une acquisition d'une image du modèle de référence actualisé, dite « image de référence », présentant une concordance maximale avec l'image actualisée dans lesdites conditions d'acquisition virtuelles.

Ce procédé permet avantageusement, après génération du modèle de référence initial, de préférence au moyen d'un scanner, d'enrichir la base d'apprentissage à différents instants actualisés, sans qu'il soit nécessaire d'effectuer un nouveau scan, et donc sans que le patient ait à se déplacer chez l'orthodontiste. Il peut en effet acquérir les images actualisées lui-même, comme décrit dans WO 2016/066651.

Un seul traitement orthodontique peut ainsi conduire à la production de centaines d'images historiques.

L'invention concerne encore un procédé d'entraînement d'un dispositif d'apprentissage profond, de préférence un réseau de neurones, comportant un enrichissement d'une base d'apprentissage selon l'invention, puis l'utilisation de ladite base d'apprentissage pour entraîner le dispositif d'apprentissage profond.

### Analyse globale

Le procédé d'analyse détaillée décrit ci-dessus permet avantageusement une analyse fine de l'image d'analyse, la situation de chaque étant de préférence évaluée.

Alternativement, le dispositif d'apprentissage profond peut être utilisé de manière globale, la base d'apprentissage contenant des images historiques dont le descriptif fournit une valeur d'attribut globale pour l'image. Autrement dit, la valeur de l'attribut d'image est relative à l'ensemble de l'image et non pas à une partie de l'image. L'attribut n'est alors pas un attribut « de dent », mais est un attribut « d'image ». Par exemple, cet attribut d'image peut définir si, au regard de l'image dans son ensemble ou d'une partie de l'image, la situation dentaire « est pathologique » ou « n'est pas pathologique », sans qu'un examen de chaque dent soit effectué. L'attribut d'image permet également de détecter, par exemple, si la bouche est ouverte ou fermée, ou, plus généralement, si l'image est adaptée à un traitement ultérieur, par exemple si elle permet de contrôler l'occlusion.

L'attribut d'image peut être en particulier relatif à
- une position et/ou une orientation et/ou une calibration d'un appareil d'acquisition utilisé pour acquérir ladite image d'analyse, et/ou
- une qualité de l'image d'analyse, et en particulier relatif à la luminosité, au contraste ou à la netteté de l'image d'analyse, et/ou
- au contenu de l'image d'analyse, par exemple à la représentation des arcades, de la langue, de la bouche, des lèvres, des mâchoires, de la gencive, d'une ou plusieurs dents ou d'un appareil dentaire, de préférence orthodontique.

Lorsque l'attribut d'image fait référence au contenu de l'image, le descriptif des images historiques de la base d'apprentissage précise une caractéristique de ce contenu. Par exemple, il peut préciser la position de la langue (par exemple « en retrait ») ou l'ouverture de la bouche du patient (par exemple bouche ouverte ou fermée) ou la présence d'une représentation d'un appareil dentaire, de préférence orthodontique, et/ou son état (par exemple appareil intact, cassé ou endommagé).

Une valeur d'attribut de dent peut être utilisée pour définir une valeur pour un attribut d'image. Par exemple, si une valeur d'un attribut de dent est « dent cariée », la valeur d'un attribut d'image peut être « situation dentaire insatisfaisante ».

L'attribut d'image peut être en particulier relatif à une situation thérapeutique.

L'invention propose un procédé d'analyse globale d'une image d'analyse d'une arcade dentaire d'un patient, ledit procédé comportant les étapes suivantes :
1') création d'une base d'apprentissage comportant plus de 1 000 images d'arcades dentaires, ou « images historiques », chaque image historique comportant une valeur d'attribut pour au moins un attribut d'image, ou « valeur d'attribut d'image » ;
2') entraînement d'au moins un dispositif d'apprentissage profond, de préférence un réseau de neurones, au moyen de la base d'apprentissage ;
3') soumission de l'image d'analyse au dispositif d'apprentissage profond de manière qu'il détermine, pour ladite image d'analyse, au moins une probabilité relative à ladite valeur d'attribut d'image, et détermination, en fonction de ladite probabilité, d'une valeur pour ledit attribut d'image pour l'image d'analyse.

L'attribut d'image peut être en particulier relatif à l'orientation de l'appareil d'acquisition lors de l'acquisition de l'image d'analyse. Il peut par exemple prendre les valeurs « photo de face », « photo de gauche » et « photo de droite ».

L'attribut d'image peut être également relatif à la qualité de l'image. Il peut par exemple prendre les valeurs « contraste insuffisant » et « contraste acceptable ».

L'attribut d'image peut être également relatif à la situation dentaire du patient, par exemple être relatif à la présence d'une carie ou à l'état d'un appareil dentaire, de préférence orthodontique, porté par le patient (« dégradé » ou « en bon état » par exemple) ou à l'adéquation de l'appareil dentaire, de préférence orthodontique, au traitement du patient (par exemple « inadapté » ou « adapté »).

L'attribut d'image peut être encore relatif à la « présence » ou « l'absence » d'un appareil dentaire, de préférence orthodontique,, ou à l'état d'ouverture de la bouche (« bouche ouverte », « bouche fermée » par exemple).

Comme on le verra plus en détail dans la suite de la description, un procédé d'analyse globale d'images selon l'invention permet avantageusement d'évaluer immédiatement et globalement le contenu de l'image d'analyse. En particulier, il est possible d'évaluer globalement une situation dentaire et, par exemple, d'en déduire la nécessité de consulter un orthodontiste.

### Définitions

Un « patient » est une personne pour laquelle un procédé selon l'invention est mis en œuvre, indépendamment du fait que cette personne suive un traitement orthodontique ou non.

Par « orthodontiste », on entend toute personne qualifiée pour prodiguer des soins dentaires, ce qui inclut également un dentiste.

Par « pièce dentaire », en particulier « pièce orthodontique », on entend tout ou partie d'un appareil dentaire, en particulier orthodontique.

Une pièce orthodontique peut être en particulier une gouttière orthodontique. Une telle gouttière s'étend de manière à suivre les dents successives de l'arcade sur laquelle elle est fixée. Elle définit une goulotte de forme générale en « U », dont la forme est déterminée pour assurer la fixation de la gouttière sur les dents, mais également en fonction d'un positionnement cible souhaité pour les dents. Plus précisément, la forme est déterminée de manière que, lorsque la gouttière est dans sa position de service, elle exerce des contraintes tendant à déplacer les dents traitées vers leur positionnement cible, ou à maintenir les dents dans ce positionnement cible.

La « position de service » est la position dans laquelle la pièce dentaire ou orthodontique est portée par le patient.

Par « modèle », on entend un modèle tridimensionnel numérique. Un agencement de modèles de dent est donc un modèle.

Par "image", on entend une image en deux dimensions, comme une photographie ou une image extraite d'un film. Une image est formée de pixels.

Une « image de référence » est une vue d'un modèle « de référence ».

Par « image d'une arcade », ou « modèle d'une arcade », on entend une représentation de tout ou partie de ladite arcade. De préférence, une telle représentation est en couleur.

Les « conditions d'acquisition » d'une image précisent la position et l'orientation dans l'espace d'un appareil d'acquisition de cette image relativement aux dents du patient (conditions d'acquisition réelles) ou à un modèle de dents du patient (conditions d'acquisition virtuelles), et de préférence la calibration de cet appareil d'acquisition. Des conditions d'acquisition sont dites "virtuelles" lorsqu'elles correspondent à une simulation dans laquelle l'appareil d'acquisition serait dans lesdites conditions d'acquisition (positionnement et de préférence calibration théoriques de l'appareil d'acquisition) par rapport à un modèle.

Dans des conditions d'acquisition virtuelles d'une image de référence, l'appareil d'acquisition peut être également qualifié de « virtuel ». L'image de référence est en effet acquise par un appareil d'acquisition fictif, ayant les caractéristiques de l'appareil d'acquisition « réel » ayant servi à l'acquisition des images réelles, et en particulier des images actualisées.

La « calibration » d'un appareil d'acquisition est constituée par l'ensemble des valeurs des paramètres de calibration. Un "paramètre de calibration" est un paramètre intrinsèque à l'appareil d'acquisition (à la différence de sa position et de son orientation) dont la valeur influence l'image acquise. De préférence, les paramètres de calibration sont choisis dans le groupe formé par l'ouverture de diaphragme, le temps d'exposition, la distance focale et la sensibilité.

Une "information discriminante" est une information caractéristique qui peut être extraite d'une image (*"image feature*"), classiquement par un traitement informatique de cette image.

Une information discriminante peut présenter un nombre variable de valeurs. Par exemple, une information de contour peut être égale à 1 ou 0 selon qu'un pixel appartient ou non à un contour. Une information de brillance peut prendre un grand nombre de valeurs. Le traitement de l'image permet d'extraire et de quantifier l'information discriminante.

L'information discriminante peut être représentée sous la forme d'une « carte ». Une carte est ainsi le résultat d'un traitement d'une image afin de faire apparaître l'information discriminante, par exemple le contour des dents et des gencives.

On appelle « concordance » (*« match »* ou *« fit » en* anglais) entre deux objets une mesure de la différence entre ces deux objets. Une concordance est maximale (« *best fit »*) lorsqu'elle résulte d'une optimisation permettant de manière à minimiser ladite différence.

Un objet modifié pour obtenir une concordance maximale peut être qualifié d'objet « optimal ».

Deux images ou « vues » qui présentent une concordance maximale représentent sensiblement au moins une même dent, de la même façon. Autrement dit, les représentations de la dent sur ces deux images sont sensiblement superposables.

La recherche d'une image de référence présentant une concordance maximale avec une image actualisée s'effectue en recherchant les conditions d'acquisition virtuelles de l'image de référence présentant une concordance maximale avec les conditions d'acquisition réelles de l'image actualisée.

Par extension, un modèle présente une concordance maximale avec une image lorsque ce modèle a été choisi parmi plusieurs modèles parce qu'il permet une vue présentant une concordance maximale avec ladite image et/ou lorsque cette image a été choisie parmi plusieurs images parce qu'elle présente une concordance maximale avec une vue dudit modèle.

En particulier, une image actualisée est en concordance maximale avec un modèle de référence lorsqu'une vue de ce modèle de référence fournit une image de référence en concordance maximale avec l'image actualisée.

La comparaison entre deux images résulte de préférence de la comparaison de deux cartes correspondantes. On appelle classiquement « « distance » une mesure de la différence entre deux cartes ou entre deux images.

Les méthodes « métaheuristiques » sont des méthodes d'optimisation connues. Elles sont de préférence choisies dans le groupe formé par
- les algorithmes évolutionnistes, de préférence choisie parmi: les stratégies d'évolution, les algorithmes génétiques, les algorithmes à évolution différentielle, les algorithmes à estimation de distribution, les systèmes immunitaires artificiels, la recomposition de chemin Shuffled Complex Evolution, le recuit simulé, les algorithmes de colonies de fourmis, les algorithmes d'optimisation par essaims particulaires, la recherche avec tabous, et la méthode GRASP ;
- l'algorithme du kangourou,
- la méthode de Fletcher et Powell,
- la méthode du bruitage,
- la tunnelisation stochastique,
- l'escalade de collines à recommencements aléatoires,
- la méthode de l'entropie croisée, et
- les méthodes hybrides entre les méthodes métaheuristiques citées ci-dessus.

On appelle « descriptif » d'une image, une information notamment relative à la définition des zones de dent de cette image et aux valeurs d'attribut de dent qui leur sont associées, et/ou relative à une valeur d'attribut d'image de ladite image. Le nombre de valeurs possible pour un attribut de dent ou un attribut d'image n'est pas limité.

Une image « historique » est une image d'une arcade dentaire enrichie d'un descriptif. Les zones de dent d'une image historique sont qualifiées de « zones de dent historiques ».

Il faut interpréter "comprenant " ou "comportant " ou "présentant " de manière non restrictive, sauf indication contraire.

### Brève description des figures

D'autres caractéristiques et avantages de l'invention apparaîtront encore à la lecture de la description détaillée qui va suivre et à l'examen du dessin annexé dans lequel :

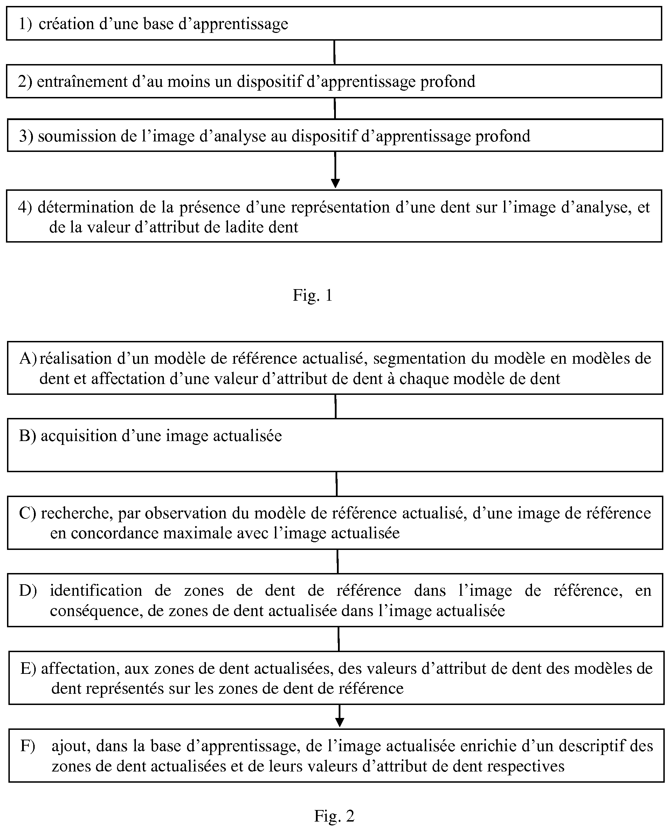

- la figure 1 représente, schématiquement, les différentes étapes d'un procédé d'analyse détaillée d'une image, selon l'invention ;
- la figure 2 représente, schématiquement, les différentes étapes d'un procédé d'enrichissement d'une base d'apprentissage, selon l'invention ;

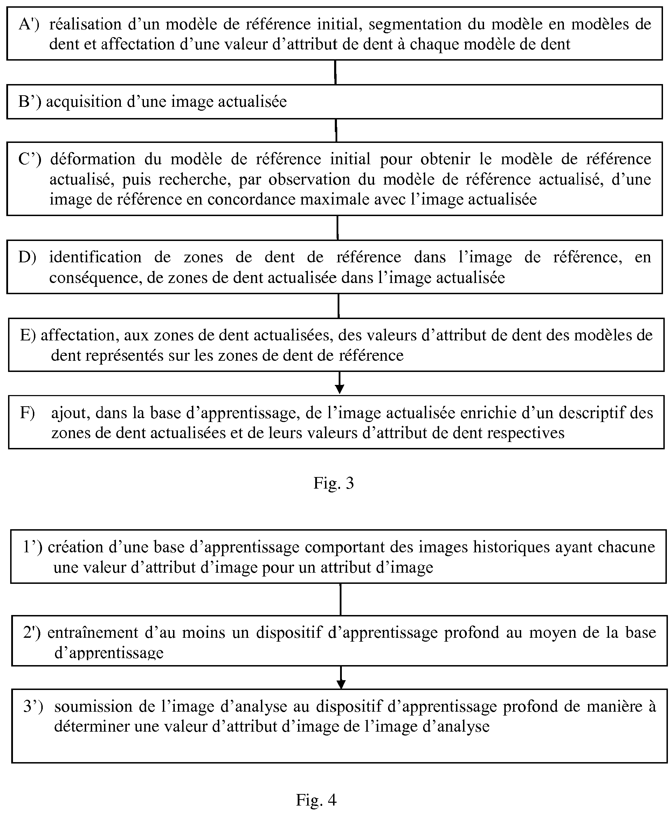

- la figure 3 représente, schématiquement, les différentes étapes d'une variante d'un procédé d'enrichissement d'une base d'apprentissage, selon l'invention ;
- la figure 4 représente, schématiquement, les différentes étapes d'un procédé d'analyse globale d'une image, selon l'invention ;

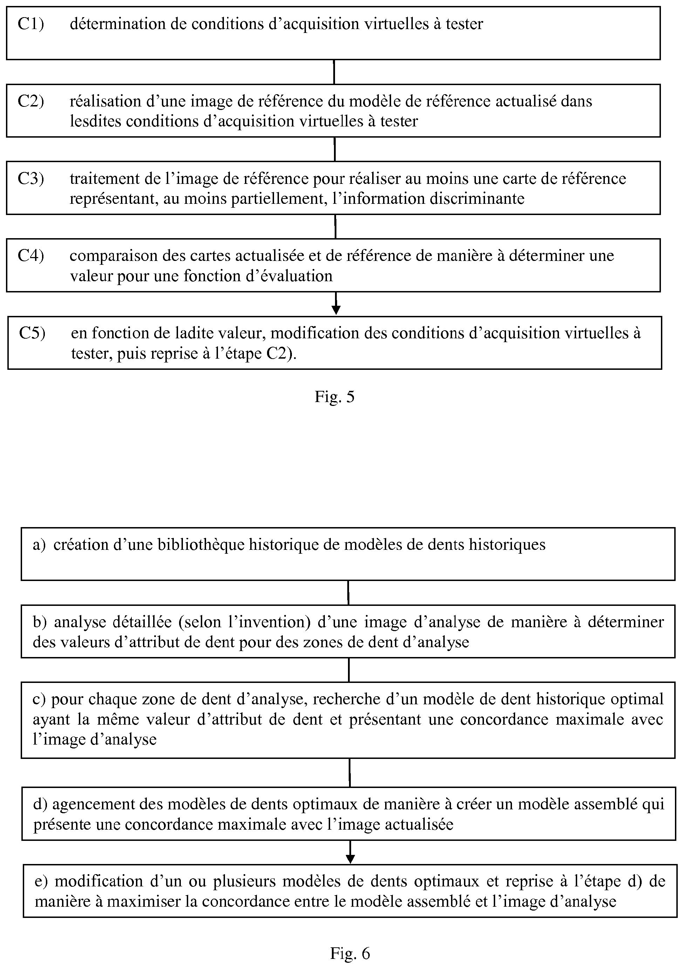

- la figure 5 représente, schématiquement, les différentes étapes d'une étape C) d'un procédé d'enrichissement d'une base d'apprentissage, selon l'invention ;
- les figures 6 et 18 représentent, schématiquement, les différentes étapes d'un procédé de modélisation de l'arcade dentaire d'un patient, selon l'invention ;

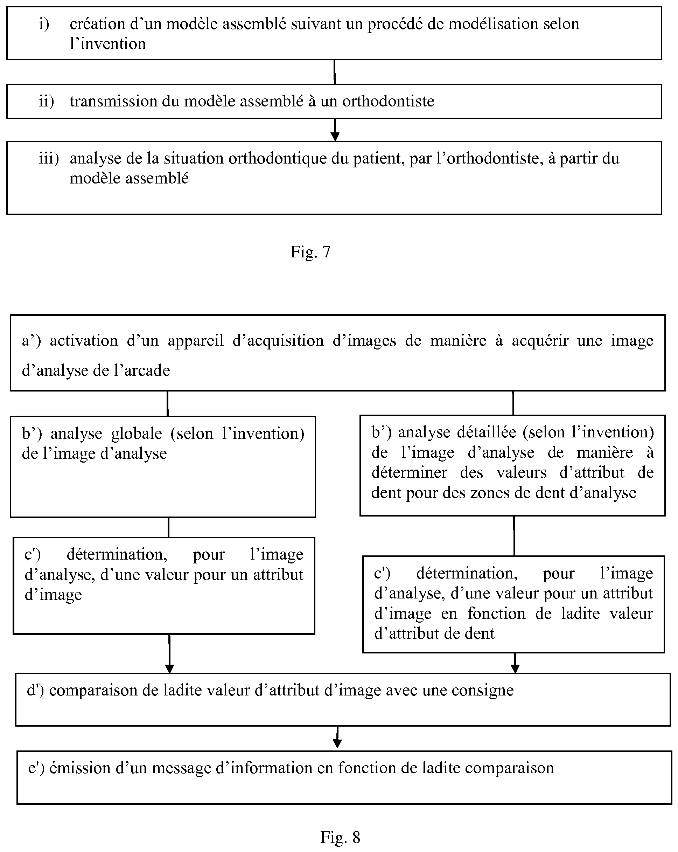

- la figure 7 représente, schématiquement, les différentes étapes d'un procédé d'évaluation d'une situation dentaire d'un patient, selon l'invention ;
- la figure 8 représente, schématiquement, les différentes étapes d'un procédé d'acquisition d'une image d'une arcade dentaire d'un patient, selon l'invention ;

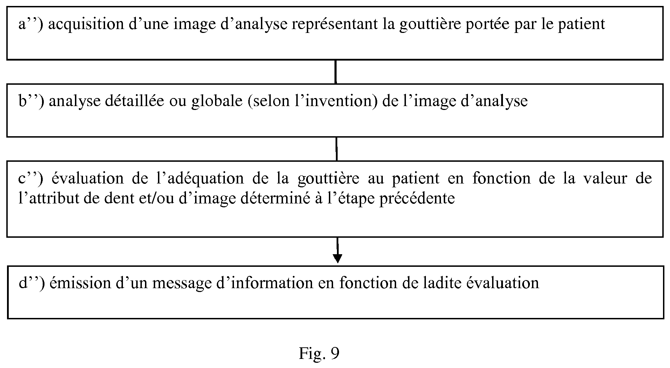

- la figure 9 représente, schématiquement, les différentes étapes d'un procédé d'évaluation de la forme d'une gouttière orthodontique d'un patient, selon l'invention ;
- la figure 10 représente un exemple d'une image de référence d'un modèle de référence initial ;
- la figure 11 (11a-11d) illustre un traitement pour déterminer les modèles de dent dans un modèle de référence initial, comme décrit dans WO 2016 066651 ;
- la figure 12 (12a-12d) illustre l'acquisition d'une image au moyen d'un écarteur, une opération de découpage de cette image, et le traitement d'une image actualisée permettant de déterminer le contour des dents, comme décrit dans WO 2016 066651 ;
- la figure 13 illustre schématiquement la position relative de marques de repérage 12 d'un écarteur 10 sur des images actualisées 14₁ et 14₂, selon les directions d'observation représentées en traits interrompus ;
- les figures 14 et 15 représentent une gouttière orthodontique, en perspective et vue de dessus, respectivement ;
- la figure 16 illustre l'étape e) décrite dans WO 2016 066651 ;
- la figure 17 illustre un procédé d'enrichissement selon l'invention.

### Description détaillée

Un procédé d'analyse détaillée selon l'invention nécessite la création d'une base d'apprentissage. Cette création met de préférence en œuvre un procédé comportant les étapes A) à F), ou, dans un mode de réalisation, à la place des étapes A) à C), de préférence les étapes A') à C').

### Premier mode de réalisation principal du procédé d'enrichissement

**L'étape A)** est destinée à la réalisation d'un modèle de référence actualisé modélisant une arcade du patient. Elle comprend de préférence une ou plusieurs des caractéristiques de l'étape a) de WO 2016 066651 pour réaliser un modèle de référence initial.

Le modèle de référence actualisé est de préférence créé avec un scanner 3D. Un tel modèle, dit « 3D », peut être observé selon un angle quelconque. Une observation du modèle, selon un angle et à une distance déterminés, est appelée une « vue » ou « image de référence ».

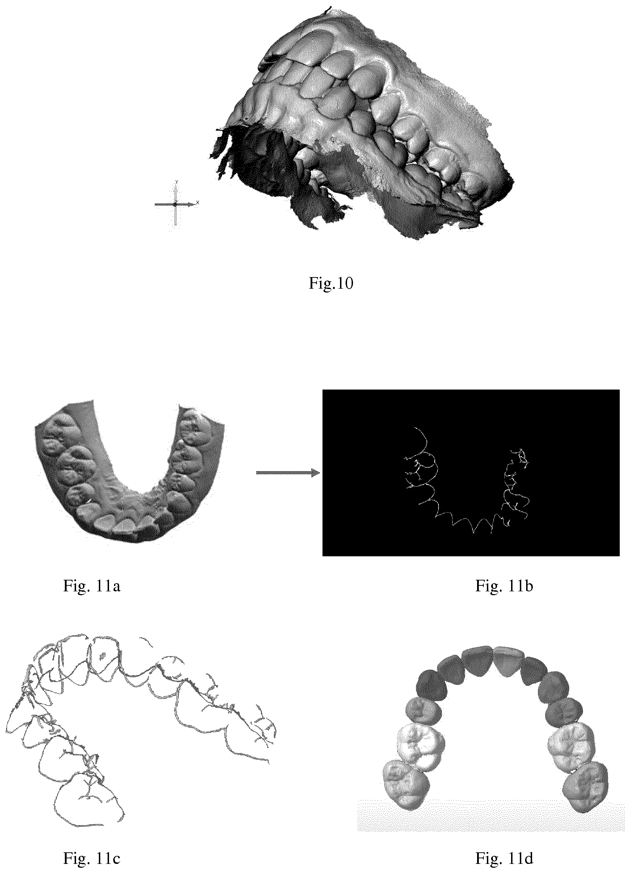

La figure 11a est un exemple d'image de référence.

Le modèle de référence actualisé peut être préparé à partir de mesures effectuées sur les dents du patient ou sur un moulage de ses dents, par exemple un moulage en plâtre.

Pour chaque dent, on définit, à partir du modèle de référence actualisé, un modèle de ladite dent, ou « modèle de dent » (figure 11d). Cette opération, connue en elle-même, est appelée « segmentation » du modèle de référence actualisé.

Dans le modèle de référence actualisé, un modèle de dent est de préférence délimité par un bord gingival qui peut être décomposé en un bord gingival intérieur (du côté de l'intérieur de la bouche par rapport à la dent), un bord gingival extérieur (orienté vers l'extérieur de la bouche par rapport à la dent) et deux bords gingivaux latéraux.

Un ou plusieurs attributs de dent sont associés aux modèles de dent en fonction des dents qu'ils modélisent.

L'attribut de dent est de préférence choisi parmi un numéro de dent, un type de dent, un paramètre de forme de la dent, par exemple une largeur de dent, en particulier une largeur mésio-palatine, une épaisseur, une hauteur de couronne, un indice de déflexion en mésial et distal du bord incisif, ou un niveau d'abrasion, un paramètre d'apparence de la dent, en particulier un indice de translucidité ou un paramètre de couleur, un paramètre relatif à l'état de la dent, par exemple « abrasée », « cassée », « cariée » ou « appareillée » (c'est-à-dire en contact avec un appareil dentaire, de préférence orthodontique), un âge pour le patient, ou une combinaison de ces attributs. Un attribut de dent est de préférence un attribut qui ne concerne que la dent modélisée par le modèle de dent.

Une valeur d'attribut de dent peut être affectée à chaque attribut de dent d'un modèle de dent particulier.

Par exemple, l'attribut de dent « type de dent » aura la valeur « incisive », « canine » ou « molaire » selon que le modèle de dent est celui d'une incisive, d'une canine ou d'une molaire, respectivement.

L'affectation des valeurs d'attribut de dent aux modèles de dent peut être manuelle ou, au moins en partie, automatique. Par exemple, si la valeur d'un attribut de dent est identique quel que soit le modèle de dent, comme pour l'attribut de dent « âge du patient », il peut suffire d'attribuer une valeur à un modèle de dent pour déterminer la valeur de cet attribut pour les autres modèles de dent.

De même, les numéros de dent sont classiquement attribués selon une règle standard. Il suffit donc de connaître cette règle et le numéro d'une dent modélisée par un modèle de dent pour calculer les numéros des autres modèles de dent.

Dans un mode de réalisation préféré, la forme d'un modèle de dent particulier est analysée de manière à définir sa valeur d'attribut de dent, par exemple son numéro. Cette reconnaissance de forme est de préférence réalisée au moyen d'un dispositif d'apprentissage profond, de préférence un réseau de neurones. De préférence, on crée une bibliothèque de modèles de dent historiques, chaque modèle de dent historique ayant une valeur pour l'attribut de dent, comme décrit ci-après (étape a)), on entraîne le dispositif d'apprentissage profond avec des vues des modèles de dent historiques de cette bibliothèque, puis on analyse une ou plusieurs vues du modèle de dent particulier avec le dispositif d'apprentissage profond entraîné, de manière à déterminer la valeur d'attribut de dent dudit modèle de dent particulier.

L'attribution des valeurs d'attribut de dent peut alors être totalement réalisée sans intervention humaine.

**L'étape B)** est destinée à l'acquisition d'une ou de préférence plusieurs images actualisées. L'étape B) comprend de préférence une ou plusieurs des caractéristiques de l'étape b) de WO 2016 066651.

L'acquisition des images actualisées est réalisée au moyen d'un appareil d'acquisition d'images, de préférence choisi parmi un téléphone mobile, un appareil photo dit « connecté », une montre dite « intelligente », ou « smartwatch », une tablette ou un ordinateur personnel, fixe ou portable, comportant un système d'acquisition d'images, comme une webcam ou un appareil photo. De préférence l'appareil d'acquisition d'images est un téléphone mobile.

De préférence encore, l'appareil d'acquisition d'images est écarté de l'arcade dentaire de plus de 5 cm, plus de 8 cm, voire plus de 10 cm, ce qui évite la condensation de vapeur d'eau sur l'optique de l'appareil d'acquisition d'images et facilite la mise au point. En outre, de préférence, l'appareil d'acquisition d'images, en particulier le téléphone mobile, n'est pourvu d'aucune optique spécifique pour l'acquisition des images actualisées, ce qui est notamment possible du fait de l'écartement de l'arcade dentaire lors de l'acquisition.

De préférence, une image actualisée est une photographie ou est une image extraite d'un film. Elle est de préférence en couleurs, de préférence en couleurs réelles.

De préférence, l'acquisition de la ou des images actualisées est effectuée par le patient, de préférence sans utilisation d'un support, prenant appui sur le sol et immobilisant l'appareil d'acquisition d'images, et notamment sans trépied.

Dans un mode de réalisation, le déclenchement de l'acquisition est automatique, c'est-à-dire sans action d'un opérateur, dès que les conditions d'acquisition sont approuvées par l'appareil d'acquisition d'images, en particulier lorsque l'appareil d'acquisition d'images a déterminé qu'il observe une arcade dentaire et/ou un écarteur et que les conditions d'observations sont satisfaisantes (netteté, luminosité, voire dimensions de la représentation de l'arcade dentaire et/ou de l'écarteur).

L'intervalle de temps entre les étapes A) et B) est le plus faible possible afin que les dents ne se soient sensiblement pas déplacées entre la réalisation du modèle actualisé et l'acquisition des images actualisées. Des images de référence concordantes avec les images actualisées peuvent alors peut être acquises en observant le modèle de référence actualisé.

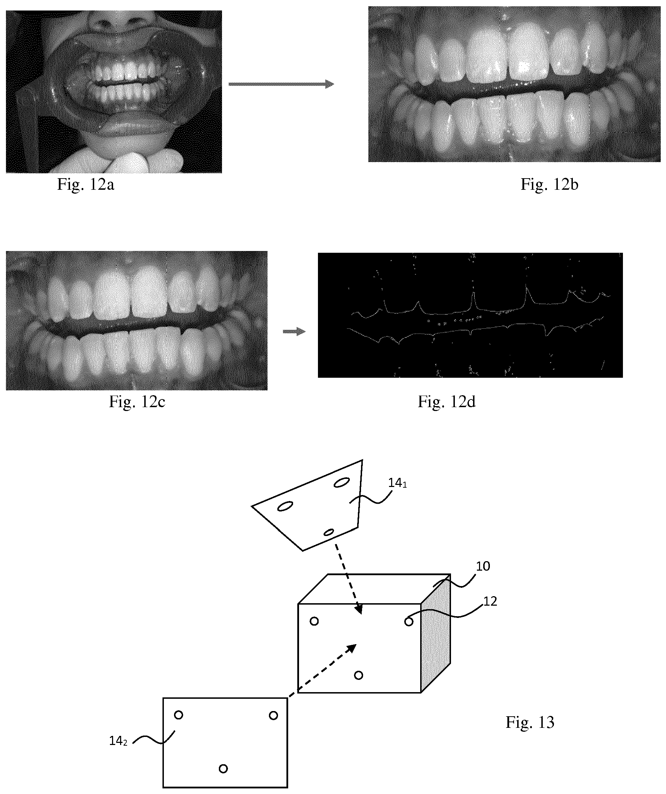

De préférence, on utilise un écarteur dentaire 10 lors de l'étape B), comme représenté sur la figure 12a. L'écarteur comporte classiquement un support muni d'un rebord s'étendant autour d'une ouverture et agencé de manière que les lèvres du patient puissent y reposer en laissant les dents du patient apparaître à travers ladite ouverture.

**A l'étape C),** le modèle de référence actualisé est exploré pour trouver, pour chaque image actualisée, une image de référence présentant une concordance maximale avec l'image actualisée.

L'étape C) peut comprendre une ou plusieurs des caractéristiques des étapes c), d) et e) de WO 2016 066651, dans la mesure où elles concernent une telle exploration.

Pour chaque image actualisée, on détermine de préférence, de manière grossière, un ensemble de conditions d'acquisition virtuelles approximant les conditions d'acquisition réelles lors de l'acquisition de ladite image actualisée. Autrement dit, on estime la position de l'appareil d'acquisition d'images par rapport aux dents au moment où il a pris l'image actualisée (position de l'appareil d'acquisition dans l'espace et orientation de cet appareil). Cette évaluation grossière permet avantageusement de limiter le nombre de tests sur des conditions d'acquisition virtuelles lors des opérations suivantes, et permet donc d'accélérer considérablement ces opérations.

Pour effectuer cette évaluation grossière, on utilise de préférence une ou plusieurs règles heuristiques. Par exemple, de préférence, on exclut des conditions d'acquisition virtuelles susceptibles d'être testées lors des opérations suivantes, les conditions qui correspondent à une position de l'appareil d'acquisition d'images derrière les dents ou à une distance des dents supérieure à 1 m.

Dans un mode de réalisation préféré, comme illustré sur la figure 13, on utilise des marques de repérage représentées sur l'image actualisée, et en particulier des marques de repérage 12 de l'écarteur, pour déterminer une région de l'espace sensiblement conique délimitant des conditions d'acquisition virtuelles susceptibles d'être testées lors des opérations suivantes, ou "cône de test".

Plus précisément, on dispose de préférence au moins trois marques de repérage 12 non alignées sur l'écarteur 10, et on mesure précisément leurs positions relatives sur l'écarteur.

Les marques de repérage sont ensuite repérées sur l'image actualisée, comme décrit précédemment. De simples calculs trigonométriques permettent de déterminer approximativement la direction selon laquelle l'image actualisée a été prise.

Ensuite, pour chaque image actualisée, on recherche une image de référence présentant une concordance maximale avec l'image actualisée. Cette recherche est de préférence effectuée au moyen d'une méthode métaheuristique, de préférence évolutionniste, de préférence par recuit simulé.

De préférence, à un instant quelconque avant l'étape C4), l'image actualisée est analysée de manière à réaliser une carte actualisée représentant, au moins partiellement, une information discriminante. La carte actualisée représente donc l'information discriminante dans le référentiel de l'image actualisée.

L'information discriminante est de préférence choisie dans le groupe constitué par une information de contour, une information de couleur, une information de densité, une information de distance, une information de brillance, une information de saturation, une information sur les reflets et des combinaisons de ces informations.

L'homme de l'art sait comment traiter une image actualisée pour faire apparaître l'information discriminante.

Par exemple, la figure 12d est une carte actualisée relative au contour des dents obtenue à partir de l'image actualisée de la figure 12b.

Ladite recherche comporte les étapes suivantes :
C1) détermination de conditions d'acquisition virtuelles « à tester » ;
C2) réalisation d'une image de référence du modèle de référence actualisé dans lesdites conditions d'acquisition virtuelles à tester ;
C3) traitement de l'image de référence pour réaliser au moins une carte de référence représentant, au moins partiellement, l'information discriminante ;
C4) comparaison des cartes actualisée et de référence de manière à déterminer une valeur pour une fonction d'évaluation, ladite valeur pour la fonction d'évaluation dépendant des différences entre lesdites cartes actualisée et de référence et correspondant à une décision de poursuivre ou de stopper la recherche de conditions d'acquisition virtuelles approximant lesdites conditions d'acquisition réelles avec plus d'exactitude que lesdites conditions d'acquisition virtuelles à tester déterminées à la dernière occurrence de l'étape C1);
C5) si ladite valeur pour la fonction d'évaluation correspond à une décision de poursuivre ladite recherche, modification des conditions d'acquisition virtuelles à tester, puis reprise à l'étape C2).

**A l'étape C1),** on commence par déterminer des conditions d'acquisition virtuelles à tester, c'est-à-dire une position et une orientation virtuelles susceptibles de correspondre à la position et l'orientation réelles de l'appareil d'acquisition lors de la capture de l'image actualisée, mais aussi, de préférence, une calibration virtuelle susceptible de correspondre à la calibration réelle de l'appareil d'acquisition lors de la capture de l'image actualisée.

Les premières conditions d'acquisition virtuelles à tester sont de préférence des conditions d'acquisition virtuelles évaluées grossièrement, comme décrit précédemment.

**A l'étape C2),** on configure ensuite virtuellement l'appareil d'acquisition d'images dans les conditions d'acquisition virtuelles à tester afin d'acquérir une image de référence du modèle de référence actualisé dans ces conditions d'acquisition virtuelles à tester. L'image de référence correspond donc à l'image qu'aurait prise l'appareil d'acquisition d'images s'il avait été placé, par rapport au modèle de référence actualisé, et optionnellement calibré, dans les conditions d'acquisition virtuelles à tester.

Si l'image actualisée a été prise sensiblement au même moment que le modèle de référence actualisé a été créé par un scan des dents du patient, la position des dents sur l'image actualisée est sensiblement identique à celle dans le modèle de référence actualisé. Si les conditions d'acquisition virtuelles à tester sont exactement les conditions d'acquisition réelles, l'image de référence est donc exactement superposable à l'image actualisée. Les différences entre l'image actualisée et l'image de référence résultent d'erreurs dans l'évaluation des conditions d'acquisition virtuelles à tester, si elles ne correspondent pas exactement aux conditions d'acquisition réelles.

**A l'étape C3),** on traite l'image de référence, comme l'image actualisée, de manière à réaliser, à partir de l'image de référence, une carte de référence représentant l'information discriminante (figures 11a et 11b). L'homme de l'art sait comment traiter une image de référence pour faire apparaître l'information discriminante.

**A l'étape C4),** les cartes actualisée et de référence, portant toutes les deux sur la même information discriminante, sont comparées et on évalue la différence ou « distance » entre ces deux cartes au moyen d'un score. Par exemple, si l'information discriminante est le contour des dents, on peut comparer la distance moyenne entre les points du contour des dents qui apparait sur l'image de référence et les points du contour correspondant qui apparaît sur l'image actualisée, le score étant d'autant plus élevé que cette distance est faible. Le score peut être par exemple un coefficient de corrélation.

De préférence, les conditions d'acquisition virtuelles comprennent les paramètres de calibration de l'appareil d'acquisition. Le score est d'autant plus élevé que les valeurs des paramètres de calibration testées sont proches des valeurs des paramètres de calibration de l'appareil d'acquisition utilisé lors de l'acquisition de l'image actualisée. Par exemple, si l'ouverture de diaphragme testée est éloignée de celle de l'appareil d'acquisition utilisé lors de l'acquisition de l'image actualisée, l'image de référence présente des régions floues et des régions nettes qui ne correspondent pas aux régions floues et aux régions nettes de l'image actualisée. Si l'information discriminante est le contour des dents, les cartes actualisée et de référence ne représenteront donc pas les mêmes contours et le score sera faible.

Le score est ensuite évalué au moyen d'une fonction d'évaluation. La fonction d'évaluation permet de décider si le cyclage sur les étapes C1) à C5) doit être poursuivi ou stoppé. La fonction d'évaluation peut par exemple être égale à 0 si le cyclage doit être stoppé ou être égale à 1 si le cyclage doit se poursuivre.

La valeur de la fonction d'évaluation peut dépendre du score atteint. Par exemple, il peut être décidé de poursuivre le cyclage si le score ne dépasse pas un seuil. Par exemple, si une correspondance exacte entre les images actualisée et de référence conduit à un score de 100%, le seuil peut être, par exemple, de 95%. Bien entendu, plus le seuil sera élevé, meilleure sera la précision de l'évaluation des conditions d'acquisition virtuelles si le score parvient à dépasser ce seuil.

La valeur de la fonction d'évaluation peut également dépendre de scores obtenus avec des conditions d'acquisition virtuelles testées précédemment.

La valeur de la fonction d'évaluation peut également dépendre de paramètres aléatoires et/ou du nombre de cycles déjà effectués.

En particulier, il est possible qu'en dépit de la répétition des cycles, on ne parvienne pas à trouver des conditions d'acquisition virtuelles qui soient suffisamment proches des conditions d'acquisition réelles pour que le score atteigne ledit seuil. La fonction d'évaluation peut alors conduire à la décision de quitter le cycle bien que le meilleur score obtenu n'ait pas atteint ledit seuil. Cette décision peut résulter, par exemple, d'un nombre de cycles supérieur à un nombre maximal prédéterminé.

Un paramètre aléatoire dans la fonction d'évaluation peut également autoriser la poursuite de tests de nouvelles conditions d'acquisition virtuelles, bien que le score apparaisse satisfaisant.

Les fonctions d'évaluation classiquement utilisées dans les procédés d'optimisation métaheuristiques, de préférence évolutionnistes, en particulier dans les procédés de recuit simulé, peuvent être utilisées pour la fonction d'évaluation.

**A l'étape C5),** si la valeur de la fonction d'évaluation indique qu'il est décidé de poursuivre le cyclage, on modifie les conditions d'acquisition virtuelles à tester et on recommence le cyclage sur les étapes C1) à C5) consistant à réaliser une image de référence et une carte de référence, à comparer la carte de référence avec la carte actualisée pour déterminer un score, puis à prendre une décision en fonction de ce score.

La modification des conditions d'acquisition virtuelles à tester correspond à un déplacement virtuel dans l'espace et/ou à une modification de l'orientation et/ou, de préférence, à une modification de la calibration de l'appareil d'acquisition. Cette modification peut être aléatoire, de préférence de manière que les nouvelles conditions d'acquisition virtuelles à tester appartiennent toujours à l'ensemble déterminé lors de l'évaluation grossière. La modification est de préférence guidée par des règles heuristiques, par exemple en favorisant les modifications qui, d'après une analyse des précédents scores obtenus, apparaissent les plus favorables pour augmenter le score.

Le cyclage est poursuivi jusqu'à ce que la valeur de la fonction d'évaluation indique qu'il est décidé de cesser ce cyclage et de poursuivre à l'étape D), par exemple si le score atteint ou dépasse ledit seuil.

L'optimisation des conditions d'acquisition virtuelles est de préférence effectuée en utilisant une méthode métaheuristique, de préférence évolutionniste, de préférence un algorithme de recuit simulé. Un tel algorithme est bien connu pour l'optimisation non linéaire.

Si on a quitté le cyclage, sans qu'un score satisfaisant ait pu être obtenu, par exemple sans que le score ait pu atteindre ledit seuil, le procédé peut être arrêté (situation d'échec) ou une nouvelle étape C) peut être lancée, avec une nouvelle information discriminante et/ou avec une nouvelle image actualisée. Le procédé peut être également poursuivi avec les conditions d'acquisition virtuelles correspondant au meilleur score atteint. Un avertissement peut être émis afin d'informer l'utilisateur de l'erreur sur le résultat.

Si on a quitté le cyclage alors qu'un score satisfaisant a pu être obtenu, par exemple parce que le score a atteint, voire dépassé ledit seuil, les conditions d'acquisition virtuelles correspondent sensiblement aux conditions d'acquisition réelles de l'image actualisée.

De préférence, les conditions d'acquisition virtuelles comprennent les paramètres de calibration de l'appareil d'acquisition. Le procédé permet ainsi d'évaluer les valeurs de ces paramètres sans qu'il soit nécessaire de connaître la nature de l'appareil d'acquisition ou son réglage. L'acquisition des images actualisées peut donc être réalisée sans précaution particulière, par exemple par le patient lui-même au moyen de son téléphone mobile.

En outre, la recherche de la calibration réelle est effectuée en comparant une image actualisée avec des vues d'un modèle de référence dans des conditions d'acquisition virtuelles à tester. Avantageusement, elle ne nécessite pas que l'image actualisée fasse apparaître une jauge étalon de calibration, c'est-à-dire une jauge dont on connaît précisément les caractéristiques permettant déterminer la calibration de l'appareil d'acquisition.

L'étape C) aboutit donc à la détermination de conditions d'acquisition virtuelles présentant une concordance maximale avec les conditions d'acquisition réelles. L'image de référence est donc en concordance maximale avec l'image actualisée, c'est-à-dire que ces deux images sont sensiblement superposables.

Dans un mode de réalisation, ladite recherche des conditions d'acquisition virtuelles à l'étape C) est effectuée en utilisant une méthode métaheuristique, de préférence évolutionniste, de préférence un algorithme de recuit simulé.

**A l'étape D),** on identifie les zones de dent de référence sur l'image de référence et on les reporte sur l'image actualisée pour définir des zones de dent actualisées correspondantes.

En particulier, l'image de référence est une vue du modèle de référence actualisé segmenté en modèles de dent. Les limites de la représentation de chaque modèle de dent sur l'image de référence, ou « zone de dent de référence », peuvent donc être identifiées.

La superposition des images actualisée et de référence permet ensuite de reporter les limites des zones de dent de référence sur l'image actualisée, et ainsi de définir les zones de dent actualisées. L'image de référence étant en concordance maximale avec l'image actualisée, les zones de dent actualisées définissent donc sensiblement les limites des modèles de dent représentés sur l'image de référence.

**A l'étape E),** on affecte, à chaque zone de dent actualisée, la ou les valeurs d'attribut de dent du modèle de dent qui lui correspond.

En particulier, l'image de référence est une vue du modèle de référence actualisé dans lequel les modèles de dent ont été affectés de valeurs d'attribut de dent respectives pour au moins un attribut de dent, par exemple un numéro de dent. Chaque zone de dent de référence peut donc hériter de la valeur d'attribut de dent du modèle de dent qu'elle représente. Chaque zone de dent actualisée peut ensuite hériter de la valeur d'attribut de dent de la zone de dent de référence qui a permis de la définir.

A l'issue de l'étape E), on obtient donc une image actualisée et un descriptif de l'image actualisée définissant une ou plusieurs zones de dent actualisées et, pour chacune de ces zones, une valeur d'attribut de dent pour au moins un attribut de dent, par exemple un numéro de dent.

On appelle « image historique » l'image actualisée enrichie de son descriptif.

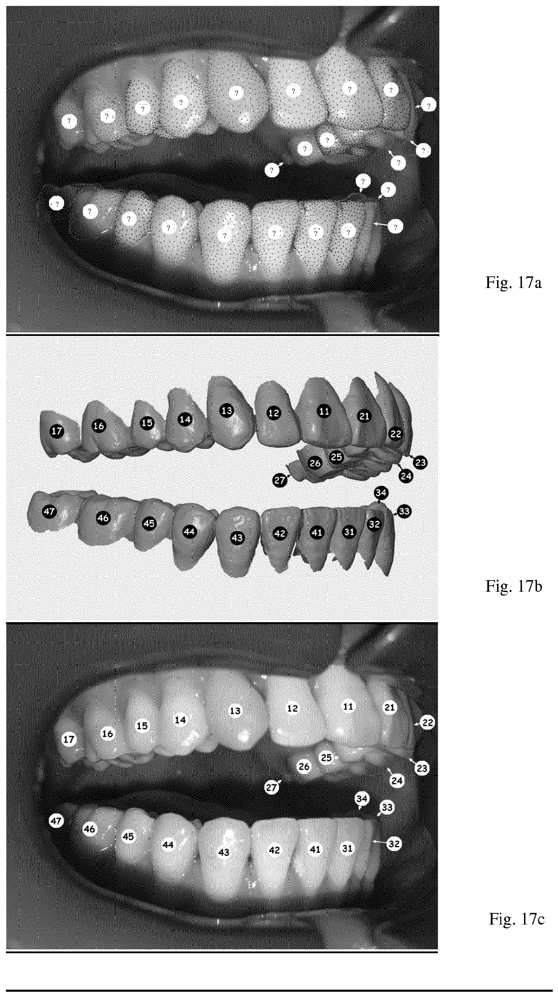

La figure 17a montre un exemple d'image actualisée (acquise lors de l'étape B)) en cours d'analyse afin de déterminer les contours des dents. La figure 17b montre l'image de référence présentant une concordance maximale avec l'image actualisée (résultant de l'étape C)). Les numéros des dents sont affichés sur les dents correspondantes. La figure 17c illustre le report des numéros de dent sur les zones de dent actualisées (étapes D) et E)).

**A l'étape F),** l'image historique est ajoutée dans la base d'apprentissage.

Les étapes A) à F) sont de préférence réalisées pour plus de 1 000, plus de 5 000, ou plus de 10 000 patients différents, ou « patients historiques ».

Comme cela apparaît clairement à présent, l'invention fournit un procédé particulièrement efficace pour créer une base d'apprentissage.

L'invention concerne également un procédé d'entrainement d'un dispositif d'apprentissage profond, de préférence un réseau de neurones, ledit procédé comportant un enrichissement d'une base d'apprentissage suivant un procédé comportant des étapes A) à F) de manière à acquérir une pluralité d'images historiques, puis l'utilisation de ladite base d'apprentissage pour entrainer ledit dispositif d'apprentissage profond.

### Deuxième mode de réalisation principal du procédé d'enrichissement

L'invention n'est cependant pas limitée aux modes de réalisation décrits ci-dessus.

En particulier, le modèle de référence actualisé n'est pas nécessairement le résultat direct d'un scan de l'arcade du patient. Le modèle de référence actualisé peut être en particulier un modèle obtenu par déformation d'un modèle de référence initial lui-même résultant directement d'un tel scan.

Le procédé comporte alors, de préférence, à la place des étapes A) à C), les étapes A') à C').

**L'étape A')** est identique à l'étape A). A l'étape A'), le modèle de référence généré est cependant destiné à être modifié. Il est donc qualifié de « modèle de référence initial », et non pas de « modèle de référence actualisé », comme selon l'étape A).

Le modèle de référence initial peut être en particulier généré à un instant initial précédant un traitement orthodontique actif, par exemple moins de 6 mois, moins de 3 mois, ou moins de 1 mois avant le début du traitement. Les étapes B') à C') peuvent être alors mises en œuvre pour suivre l'évolution du traitement entre l'instant initial et l'instant actualisé de l'étape B').

L'instant initial peut être alternativement un instant en fin de traitement orthodontique actif, par exemple moins de 6 mois, moins de 3 mois, ou moins de 1 mois après la fin du traitement. Les étapes B') à C') peuvent alors être mises en œuvre pour surveiller l'apparition d'une récidive éventuelle.

**L'étape B')** est identique à l'étape B). A l'étape B'), les images actualisées sont cependant également destinées à guider la modification du modèle de référence initial pour définir le modèle de référence actualisé, à l'étape C').

L'intervalle de temps entre les étapes A') et B') n'est limité, puisque, comme expliqué ci-après, le modèle de référence initial sera déformé pour obtenir un modèle de référence actualisé en concordance maximale avec les images actualisées. L'intervalle de temps entre les étapes A') et B') peut être par exemple supérieur à 1 semaine, à 2 semaines, à 1 mois, à 2 mois ou à 6 mois.

**L'étape C')** est plus complexe que l'étape C) puisque la recherche d'une image de référence présentant une concordance maximale avec une image actualisée ne se limite pas à rechercher les conditions d'acquisition virtuelles optimales. Elle comprend également une recherche d'un modèle de référence actualisé, c'est-à-dire d'un modèle de référence dans lequel les dents ont sensiblement la même position que sur l'image actualisée.

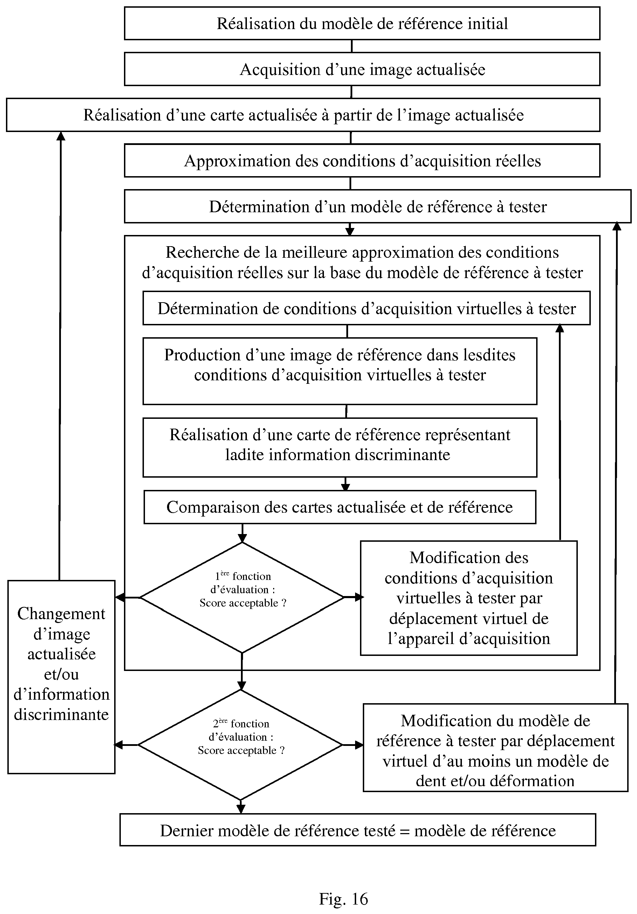

L'étape C') comprend de préférence une ou plusieurs des caractéristiques des étapes c), d) et e) de WO 2016 066651, et en particulier de l'étape e) illustrée sur la figure 16.

L'objectif est de modifier le modèle de référence initial jusqu'à obtenir un modèle de référence actualisé qui présente une concordance maximale avec l'image actualisée. Idéalement, le modèle de référence actualisé est donc un modèle de l'arcade à partir duquel l'image actualisée aurait pu être prise si ce modèle avait été l'arcade elle-même.

On teste donc une succession de modèles de référence « à tester », le choix d'un modèle de référence à tester étant de préférence dépendant du niveau de correspondance des modèles de référence « à tester » précédemment testés avec l'image actualisée.

De préférence, la recherche comporte, pour une image actualisée,
- une première opération d'optimisation permettant de rechercher, dans un modèle de référence à tester déterminé à partir du modèle de référence initial, des conditions d'acquisition virtuelles correspondant au mieux aux conditions d'acquisition réelles de l'image actualisée, et
- une deuxième opération d'optimisation permettant de rechercher, en testant une pluralité de dits modèles de référence à tester, le modèle de référence correspondant au mieux au positionnement des dents du patient lors de l'acquisition de l'image actualisée.

De préférence, une première opération d'optimisation est effectuée pour chaque test d'un modèle de référence à tester lors de la deuxième opération d'optimisation.

De préférence, la première opération d'optimisation et/ou la deuxième opération d'optimisation, de préférence la première opération d'optimisation et la deuxième opération d'optimisation mettent en œuvre une méthode métaheuristique, de préférence évolutionniste, de préférence un recuit simulé.

L'étape C') aboutit donc à la détermination
- d'un modèle de référence actualisé présentant une concordance maximale avec l'image actualisée, et
- de conditions d'acquisition virtuelles présentant une concordance maximale avec les conditions d'acquisition réelles.

Un procédé comportant les étapes A') à C') peut être avantageusement mis en œuvre dans le cadre d'un traitement orthodontique actif ou passif, ou, de manière plus générale, pour suivre toute évolution des dents.

Dans les différents procédés selon l'invention, l'enrichissement de la base d'apprentissage ne résulte pas nécessairement d'un procédé d'enrichissement selon l'invention.

Dans un mode de réalisation, la base d'apprentissage est créée par un opérateur. Ce dernier analyse ainsi des milliers d'images d'analyse. Pour que la base d'apprentissage puisse servir à la mise en œuvre d'un procédé d'analyse détaillée, il détermine les zones de dent, puis leur attribue des valeurs d'attribut de dent. Pour que la base d'apprentissage puisse servir à la mise en œuvre d'un procédé d'analyse globale, il attribue des valeurs d'attribut d'image à chaque image. Il peut ainsi constituer des images historiques.

### Procédé d'analyse détaillée d'images

Le procédé d'analyse détaillée d'une « image d'analyse » d'une arcade dentaire d'un patient selon l'invention comporte les étapes 1) à 4).

De préférence, l'image d'analyse, de préférence une photographie ou une image extraite d'un film, de préférence en couleurs, de préférence en couleurs réelles, est acquise avec un appareil d'acquisition d'images, de préférence un téléphone mobile, écarté de l'arcade dentaire de plus de 5 cm, plus de 8 cm, voire plus de 10 cm, et qui, de préférence n'est pourvu d'aucune optique spécifique.

De préférence, l'image d'analyse représente plusieurs dents, de préférence plus de 2, plus de 3, plus de 4 ou plus de 5 dents du patient.

La figure 12a ou la figure 12b pourraient être des exemples d'images d'analyse. La disposition des dents y est réaliste, c'est-à-dire qu'elle correspond à celle qu'observée par l'appareil d'acquisition d'images lorsqu'il a acquis l'image d'analyse.

De préférence, l'acquisition de l'image d'analyse est effectuée par le patient, de préférence sans utilisation d'un support, prenant appui sur le sol et immobilisant l'appareil d'acquisition d'images, et notamment sans trépied.

Dans un mode de réalisation, le déclenchement de l'acquisition de l'image d'analyse est automatique, c'est-à-dire sans action d'un opérateur, dès que les conditions d'acquisition sont approuvées par l'appareil d'acquisition d'images, en particulier lorsque l'appareil d'acquisition d'images a déterminé qu'il observe une arcade dentaire et/ou un écarteur et que les conditions d'observations sont satisfaisantes (netteté, luminosité, voire dimensions de la représentation de l'arcade dentaire et/ou de l'écarteur).

**A l'étape 1),** on crée une base d'apprentissage comportant plus de 1 000, de préférence plus de 5 000, de préférence plus de 10 000, de préférence plus de 30 000, de préférence plus de 50 000, de préférence plus de 100 000 images historiques. Plus le nombre d'images historiques est élevé, meilleure est l'analyse réalisée par le procédé.

De préférence on utilise une base d'apprentissage enrichie suivant un procédé d'enrichissement selon l'invention.

La base d'apprentissage peut cependant être constituée suivant d'autres procédés, par exemple être créée manuellement. Pour créer une image historique de la base d'apprentissage, un opérateur, de préférence un orthodontiste, identifie une ou plusieurs zones de dent « historiques » sur une image, dite puis affecte, à chaque zone de dent historique identifiée, une valeur pour au moins un attribut de dent
**A l'étape 2),** on entraîne un dispositif d'apprentissage profond, de préférence un réseau de neurones, avec la base d'apprentissage.

Un « réseau de neurones » ou « réseau neuronal artificiel » est un ensemble d'algorithmes bien connu de l'homme de l'art.

Le réseau de neurones peut être en particulier choisi parmi :
- les réseaux spécialisés dans la classification d'images, appelés « CNN » (« Convolutional neural network »), par exemple
   - AlexNet (2012)
   - ZF Net (2013)
   - VGG Net (2014)
   - GoogleNet (2015)
   - Microsoft ResNet (2015)
   - Caffe: BAIR Reference CaffeNet, BAIR AlexNet
   - Torch:VGG_CNN_S,VGG_CNN_M,VGG_CNN_M_2048,VGG_CNN_M_1 0 24,VGG_CNN_M_128,VGG_CNN_F,VGG ILSVRC-2014 16-layer,VGG ILSVRC-2014 19-layer,Network-in-Network (Imagenet & CIFAR-10)
   - Google : Inception (V3, V4).
- Les réseaux spécialisés dans la localisation, et détection d'objets dans une image, les Object Detection Network, par exemple:
   - R-CNN (2013)
   - SSD (Single Shot MultiBox Detector : Object Detection network), Faster R-CNN (Faster Region-based Convolutional Network method : Object Detection network)
   - Faster R-CNN (2015)
   - SSD (2015).

La liste ci-dessus n'est pas limitative.

A l'étape 2), le dispositif d'apprentissage profond est de préférence entrainé par un processus d'apprentissage appelé *« deep learning ».* En présentant, en entrée du dispositif d'apprentissage profond, des images historiques (images + descriptifs), le dispositif d'apprentissage profond apprend progressivement à reconnaître sur une image, des motifs, en anglais *« patterns »,* et à les associer à des zones de dent et à des valeurs d'attribut de dent, par exemple des numéros de dent.

**A l'étape 3),** on soumet l'image que l'on souhaite analyser, ou « image d'analyse », au dispositif d'apprentissage profond.

Grâce à son entrainement à l'étape 2), le dispositif d'apprentissage profond est capable d'analyser l'image d'analyse et d'y reconnaître desdits motifs. Il peut en particulier déterminer une probabilité relative à :
- la présence, à un emplacement dans ladite image d'analyse, d'une zone représentant, au moins partiellement, une dent, ou « zone de dent d'analyse »,
- la valeur d'attribut de la dent représentée sur ladite zone de dent d'analyse.

Par exemple, il est capable de déterminer qu'il y a 95% de chances qu'une forme de l'image d'analyse représente une incisive.

De préférence, le dispositif d'apprentissage profond analyse toute l'image d'analyse et détermine des probabilités pour l'ensemble des zones de dent d'analyse qu'il a identifiées.

**A l'étape 4),** on analyse les résultats de l'étape précédente pour déterminer les dents représentées sur l'image d'analyse.

Lorsque la base d'apprentissage comporte plus de 10 000 images historiques, l'étape 3) conduit à des résultats particulièrement satisfaisants. En particulier, une telle base d'apprentissage permet d'établir un seuil de probabilité telle que si une probabilité associée à une zone de dent d'analyse et à une valeur d'attribut de dent pour cette zone de dent d'analyse dépasse ledit seuil, la zone de dent d'analyse représente effectivement une dent ayant ladite valeur d'attribut de dent.

L'étape 4) conduit ainsi à la définition d'une image d'analyse enrichie d'un descriptif définissant les zones de dent d'analyse et, pour chaque zone de dent d'analyse, les valeurs des attributs de la dent représentée par la zone de dent d'analyse.

### Procédé d'analyse globale d'images

Le procédé d'analyse globale d'une image actualisée d'une arcade dentaire d'un patient selon l'invention comporte les étapes 1') à 3').

Le procédé est similaire au procédé d'analyse détaillée décrit ci-dessus, à la différence que, selon l'analyse globale, il n'est pas nécessaire d'analyser la situation individuelle de chaque dent. L'analyse est globale à toute l'image. Autrement dit, le dispositif d'apprentissage profond détermine la valeur d'un attribut « d'image » sans devoir déterminer préalablement des valeurs d'attribut de dent.

Par exemple, le dispositif d'apprentissage profond peut conclure que, « globalement », la situation dentaire est « satisfaisante » ou « insatisfaisante », sans déterminer la dent éventuellement à l'origine de l'insatisfaction.

**L'étape 1')** est similaire à l'étape 1). Les images historiques comportent cependant un descriptif précisant une valeur d'attribut d'image pour chaque image.

**L'étape 2')** est similaire à l'étape 2).

**A l'étape 3'),** on soumet l'image d'analyse au dispositif d'apprentissage profond.

Grâce à son entrainement à l'étape 2'), le dispositif d'apprentissage profond est capable d'analyser l'image d'analyse et d'y reconnaître desdits motifs. En fonction de ces motifs, il peut en particulier déterminer une probabilité relative à la valeur de l'attribut d'image considéré.

### Application à la modélisation d'une arcade dentaire

Un procédé d'analyse détaillée selon l'invention est en particulier utile pour modéliser une arcade dentaire, notamment pour l'établissement d'un diagnostic à distance.

Il est souhaitable que chacun fasse régulièrement contrôler sa dentition, notamment afin de vérifier que la position de ses dents n'évolue pas défavorablement. Lors d'un traitement orthodontique, cette évolution défavorable peut notamment conduire à modifier le traitement. Après un traitement orthodontique, une évolution défavorable, appelée « récidive », peut conduire à une reprise d'un traitement. Enfin, de manière plus générale et indépendamment de tout traitement, chacun peut souhaiter suivre les déplacements éventuels de ses dents.

Classiquement, les contrôles sont effectués par un orthodontiste qui dispose d'un appareillage adapté. Ces contrôles sont donc coûteux. En outre, les visites sont contraignantes. Enfin, certaines personnes appréhendent une visite chez un orthodontiste et renonceront à prendre rendez-vous pour un simple contrôle ou pour évaluer la faisabilité d'un traitement orthodontique.

US 2009/0291417 décrit un procédé permettant de créer, puis de modifier des modèles tridimensionnels, notamment pour la fabrication d'appareils orthodontiques.

WO 2016 066651 décrit un procédé de contrôle du positionnement et/ou de la forme et/ou de l'aspect de dents d'un patient. Ce procédé comporte une étape de création d'un modèle de référence initial des dents, à un instant initial, de préférence avec un scanner 3D, puis, à un instant ultérieur, ou « instant actualisé », par exemple six mois après l'instant initial, la création d'un modèle de référence actualisé, par déformation du modèle de référence initial. Cette déformation est effectuée de manière que le modèle de référence actualisé permette des observations sensiblement identiques à des images des dents acquises à l'instant actualisé, en particulier à des photos ou des images d'une vidéo prises par le patient lui-même, sans précautions particulières, dites « images actualisées ».

Les images actualisées servent donc à modifier le modèle de référence initial, très précis. Le modèle de référence actualisé qui résulte de la déformation du modèle de référence initial, guidée par l'analyse des images actualisées, est donc également très précis.

Le procédé décrit dans WO 2016/66651 nécessite cependant un rendez-vous chez l'orthodontiste afin de créer le modèle de référence initial. Ce rendez-vous constitue un frein à la prévention. En effet, un patient ne consultera pas nécessairement un orthodontiste s'il n'en perçoit pas la nécessité. Autrement dit, le procédé n'est souvent mis en œuvre que lorsqu'une malocclusion est constatée et qu'il faut la corriger.

Il existe donc un besoin pour un procédé répondant à ce problème, en facilitant la prévention.

Un but de l'invention est de répondre à ce besoin.

A cet effet, l'invention propose un procédé de modélisation d'une arcade dentaire d'un patient, ledit procédé comportant les étapes suivantes :
a) création d'une bibliothèque historique comportant plus de 1 000 modèles de dent, dits « modèles de dent historiques », et attribution à chaque modèle de dent historique, d'une valeur pour au moins un attribut de dent, ou « valeur d'attribut de dent » ;
b) analyse d'au moins une « image d'analyse » de l'arcade dentaire suivant un procédé d'analyse détaillée selon l'invention, de manière à déterminer au moins une zone de dent d'analyse et au moins une valeur d'attribut de dent associée à ladite zone de dent d'analyse ;
c) pour chaque zone de dent d'analyse déterminée à l'étape précédente, recherche, dans la bibliothèque historique, d'un modèle de dent historique présentant une proximité maximale avec l'image d'analyse ou avec la zone de dent d'analyse, ou « modèle de dent optimal » ;
d) agencement de l'ensemble des modèles de dent optimaux de manière à créer un modèle qui présente une concordance maximale avec l'image actualisée, ou « modèle assemblé » ;
e) optionnellement, remplacement d'au moins un modèle de dent optimal par un autre modèle de dent historique et reprise à l'étape d) de manière à maximiser la concordance entre le modèle assemblé et l'image d'analyse ;
f) optionnellement, reprise de l'étape b) avec une autre image d'analyse et à l'étape d) et/ou e), recherche d'une concordance maximale avec l'ensemble des images d'analyse utilisées.

L'invention permet ainsi, à partir d'une simple image d'analyse, par exemple d'une photographie prise au moyen d'un téléphone mobile, de reconstituer, avec une bonne fiabilité, une arcade dentaire sous la forme d'un modèle assemblé.

L'image d'analyse peut être en particulier acquise comme décrit à l'étape 1) ci-dessus.

Bien entendu, l'analyse d'une seule image d'analyse ne suffit pas à générer un modèle assemblé qui corresponde précisément à l'agencement des dents du patient. Une telle précision n'est cependant généralement pas indispensable pour effectuer un premier diagnostic de la situation dentaire du patient.

Par ailleurs, la précision du modèle assemblé peut être augmentée si plusieurs images d'analyse sont traitées.

Les étapes b) à c) sont de préférence mises en œuvre pour plusieurs images d'analyse et, aux étapes d) et e), on recherche des modèles de dent optimaux et un modèle assemblé de manière à obtenir une concordance maximale au regard de l'ensemble des images d'analyse (étape f)).

L'invention concerne encore un procédé d'évaluation d'une situation dentaire d'un patient, comportant les étapes suivantes :
i) création d'un modèle assemblé suivant un procédé de modélisation selon l'invention ;
ii) transmission du modèle assemblé à un destinataire, de préférence un orthodontiste et/ou un ordinateur ;
iii) analyse de la situation orthodontique du patient, par le destinataire, à partir du modèle assemblé ;
iv) de préférence, information du patient de la situation orthodontique, de préférence, par l'intermédiaire de son téléphone mobile.

Le patient peut donc demander très facilement à un orthodontiste de vérifier sa situation dentaire, sans même devoir se déplacer, en se contentant de transmettre une ou de préférence plusieurs photos de ses dents.

Un procédé de modélisation est à présent décrit en détail.

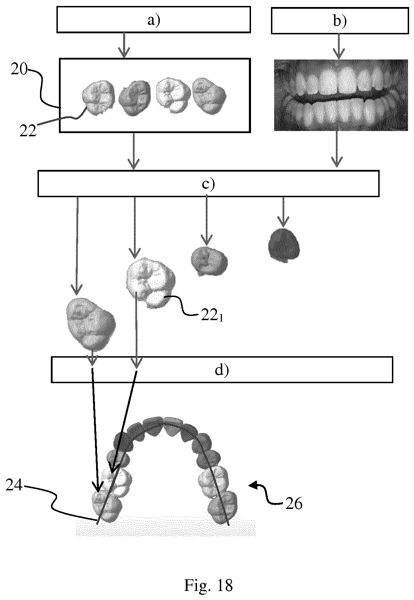

**A l'étape a),** on crée une bibliothèque historique 20 (figure 18) comportant plus de 1 000, de préférence plus de 5 000, de préférence plus de 10 000 modèles de dent historiques 22. Plus le nombre de modèles de dent historiques est élevé, plus précis est le modèle assemblé.

Un modèle de dent historique peut être en particulier obtenu à partir d'un modèle d'une arcade dentaire d'un patient « historique » obtenu avec un scanner. Ce modèle d'arcade peut être segmenté afin d'isoler les représentations des dents, comme sur la figure 11d. Chacune de ces représentations, présentant une nuance de gris spécifique sur la figure 11d, peut constituer un modèle de dent historique.

De préférence, on enrichit la bibliothèque avec les modèles de dent résultant de la mise en œuvre du procédé décrit dans WO 2016 066651 ou de l'étape A) ou A') décrite ci-dessus.

Un ou plusieurs attributs de dent, en particulier choisi dans la liste fournie ci-dessus, sont associés aux modèles de dent. Une valeur d'attribut de dent est affectée à chaque attribut de dent d'un modèle de dent particulier, comme décrit précédemment (voir la description de l'étape A)). Par exemple, un modèle de dent est celui d'une « incisive », « fortement usée » et dont les paramètres de couleur sont, dans le système de couleurs L*a*b* selon la norme NF ISO 7724, « a*=2 », « b*=1 » et « L*=58 ».

La bibliothèque historique contient donc des modèles de dent historiques et des valeurs d'attribut associées qui facilitent la recherche à l'étape c). Sur la figure 18, seuls des modèles de dent historiques 22 représentant des molaires ont été représentés dans la bibliothèque historique 20.

**A l'étape b),** on acquiert l'image d'analyse, comme décrit ci-dessus pour l'étape B), avant de l'analyser. En particulier, l'image d'analyse est de préférence une photo ou une image d'un film, de préférence avec un téléphone mobile.

L'image d'analyse peut être acquise à tout moment après l'étape a), par exemple plus de 1 semaine, plus de 1 mois ou plus de 6 mois après l'étape a).

L'image d'analyse est analysée suivant un procédé d'analyse détaillée selon l'invention. Les caractéristiques optionnelles de ce procédé sont également optionnelles à l'étape b).

A l'issue de l'étape b), on obtient une image d'analyse enrichie d'un descriptif fournissant, pour chaque zone de dent d'analyse, une valeur d'attribut de dent pour au moins un attribut de dent, par exemple un numéro de dent.

**A l'étape c),** on recherche dans la bibliothèque historique, pour chaque zone de dent d'analyse déterminée à l'étape précédente, un modèle de dent historique présentant une proximité maximale avec la zone de dent d'analyse. Ce modèle de dent est qualifié de « modèle de dent optimal ».

La « proximité » est une mesure d'une ou plusieurs différences entre le modèle de dent historique et la zone de dent d'analyse. Ces différences peuvent inclure une différence de forme, mais également d'autres différences comme une différence de translucidité ou de couleur. On peut rechercher la proximité maximale en minimisant successivement plusieurs différences, ou en minimisant une combinaison de ces différences, par exemple une somme pondérée de ces différences.

La « proximité » est donc une notion plus large que la « concordance », la concordance ne mesurant qu'une proximité relative à la forme.

L'évaluation de la proximité d'un modèle de dent historique avec une zone de dent d'analyse comporte de préférence une comparaison d'au moins une valeur d'un attribut de dent de la zone de dent d'analyse avec la valeur de cet attribut pour le modèle de dent historique. Une telle évaluation est avantageusement très rapide.

Par exemple, si le descriptif de la zone de dent d'analyse fournit une valeur pour le type ou le numéro de la dent, de l'épaisseur de la dent représentée et/ou la hauteur de sa couronne et/ou sa largeur mésio-palatine et/ou l'indice de déflexion en mésial et distal de son bord incisif, cette valeur peut être comparée à la valeur de l'attribut correspondant de chacun des modèles de dent historiques.

De préférence, on recherche un modèle de dent historique ayant, pour au moins un attribut de dent, la même valeur que ladite zone de dent d'analyse. L'attribut de dent peut en particulier être relatif au type de dent ou au numéro de dent. Autrement dit, on filtre les modèles de dent historique pour n'examiner plus en détail que ceux qui sont relatifs au même type de dent que la dent représentée sur la zone de dent d'analyse.

Alternativement ou, de préférence, en complément de cette comparaison des valeurs d'attribut, la forme de la dent représentée sur la zone de dent d'analyse peut être comparée à la forme d'un modèle de dent historique à évaluer, de préférence au moyen d'une méthode métaheuristique, de préférence évolutionniste, de préférence par recuit simulé.

A cet effet, on observe suivant différents angles le modèle de dent historique à évaluer. Chaque vue ainsi obtenue est comparée avec l'image d'analyse, de préférence avec la zone de dent d'analyse de manière à établir une « distance » entre cette vue et ladite image d'analyse ou, de préférence, ladite zone de dent d'analyse. La distance mesure ainsi la différence entre la vue et la zone de dent d'analyse.

La distance peut être déterminée après un traitement de la vue et de l'image d'analyse ou, de préférence, de la zone de dent d'analyse, de manière à faire apparaître, sur des cartes correspondantes, une même information discriminante, par exemple une information de contour, comme décrit ci-dessus à l'étape C3) ou dans WO 2016 066651.

Pour chaque modèle de dent historique testé, on détermine ainsi une vue fournissant une distance minimale avec l'image d'analyse ou avec la zone de dent d'analyse. Chaque modèle de dent historique examiné est ainsi associé à une distance minimale particulière, qui mesure sa proximité de forme avec la zone de dent d'analyse.

Le modèle de dent historique optimal est celui qui, au regard de la ou des comparaisons effectuées, est considéré comme le plus proche de la zone de dent d'analyse.

On compare ensuite les distances minimales obtenues pour les différents modèles de dent testés et on retient, pour définir le modèle de dent optimal, celui qui présente la plus petite distance minimale. Le modèle de dent optimal présente donc une concordance maximale avec l'image d'analyse.

La recherche de la concordance maximale s'effectue de préférence au moyen d'une méthode métaheuristique, de préférence évolutionniste, de préférence par recuit simulé.

Dans un mode de réalisation préféré, on effectue successivement une première évaluation des modèles de dent historiques par comparaison des valeurs d'au moins un attribut de dent, par exemple du numéro de dent, avec les valeurs correspondantes de la zone de dent d'analyse, puis une deuxième évaluation par comparaison de forme. La première évaluation, rapide, permet avantageusement de filtrer les modèles de dent historiques afin de ne soumettre à la deuxième évaluation, plus lente, que les modèles de dent historiques retenus par la première évaluation.

Par exemple, si une zone de dent d'analyse représente une dent n°15, la première évaluation permet de ne retenir que les modèles de dent modélisant des dents n°15. Lors de la deuxième évaluation, on recherche, parmi l'ensemble des modèles de dent historiques modélisant des dents n°15, le modèle de dent historique dont la forme se rapproche le plus de celle de la dent représentée.

De préférence encore, plusieurs premières évaluations sont effectuées avant d'effectuer la deuxième évaluation. Par exemple, les premières évaluations permettent de filtrer les modèles de dent historiques de manière à ne retenir que les modèles de dent modélisant des dents n°15 et qui présentent une hauteur de couronne comprise entre 8 et 8,5 mm.

A l'issue de l'étape c), on a ainsi associé un modèle de dent optimal à chacune des zones de dent d'analyse.

Par exemple, sur la figure 18, le modèle de dent historique 22₁ peut être observé de manière à ressembler fortement à une zone d'analyse identifiée sur l'image d'analyse. Il est considéré comme optimal pour cette zone d'analyse.

**A l'étape d),** on crée un modèle assemblé en agençant les modèles de dent optimaux.

Suivant un mode de réalisation, au début de l'étape d), on crée un premier agencement grossier, c'est-à-dire que l'on fabrique un modèle grossier par assemblage des modèles de dent optimaux.

Pour établir le premier agencement grossier, on peut orienter les modèles de dent optimaux de manière que leurs directions d'observation optimales soient toutes parallèles, la direction d'observation optimale d'un modèle de dent étant la direction selon laquelle ledit modèle de dent présente une concordance maximale avec l'image d'analyse.

Le premier agencement grossier peut être également établi en considérant les valeurs d'attribut de dent des modèles de dent optimaux. Par exemple, si les numéros de dent de modèles de dent optimaux sont ceux des canines et des incisives, ces modèles de dent peuvent être disposés suivant un arc 24 (figure 18) correspondant classiquement à la région de l'arcade qui porte ces types de dents.

La forme de cet arc peut être affinée en fonction d'autres valeurs d'attribut de dent.

L'ordre des modèles de dent optimaux est celui des zones de dent d'analyse correspondantes.

Par ailleurs, la distance minimale associée à un modèle de dent optimal résulte d'une observation du modèle de dent suivant une direction d'observation « optimale ». Autrement dit, c'est vraisemblablement sensiblement selon cette direction que la dent que ce modèle modélise est également observée dans l'image d'analyse. Tous les modèles de dent optimaux sont ainsi de préférence orientés de manière que leurs directions d'observation optimales respectives soient toutes parallèles.

Il est ainsi possible de définir un premier agencement des modèles de dent optimaux.

De préférence, le premier agencement des modèles de dent optimaux est ensuite modifié itérativement, de manière à présenter une concordance maximale avec l'image d'analyse.

Pour évaluer un agencement, on l'observe suivant différents angles. Chaque vue ainsi obtenue est comparée avec l'image d'analyse de manière à établir une « distance » entre cette vue et ladite image d'analyse. La distance mesure ainsi la différence entre la vue et l'image d'analyse.

La distance peut être déterminée après un traitement de la vue et de l'image d'analyse de manière à faire apparaître, sur une des cartes correspondantes, une information discriminante, par exemple une information de contour, comme décrit ci-dessus à l'étape C3) ou dans WO 2016 066651.

Pour chaque agencement examiné, on détermine ainsi une vue fournissant une distance minimale avec l'image d'analyse. Chaque agencement examiné est ainsi associé à une distance minimale.

On compare ensuite les distances minimales obtenues pour les différents agencements testés et on retient, pour définir l'agencement optimal, celui qui présente la plus petite distance minimale. L'agencement optimal présente donc une concordance maximale avec l'image d'analyse.

La recherche de la concordance maximale s'effectue de préférence au moyen d'une méthode métaheuristique, de préférence évolutionniste, de préférence par recuit simulé.

A l'issue de l'étape d), on obtient un agencement optimal des modèles de dent optimaux, c'est-à-dire le modèle assemblé 26.

**A l'étape e)** optionnelle, on remplace un ou plusieurs modèles de dent optimaux par d'autres modèles de dent, puis on reprend à l'étape d) de manière à maximiser la concordance entre le modèle assemblé et l'image d'analyse.

Il est en effet possible qu'un modèle de dent optimal, dans l'agencement « optimal », ne présente plus une concordance maximale avec l'image d'analyse. En particulier, le modèle de dent pouvait être observé dans une direction « optimale » qui fournissait une vue présentant une distance minimale avec l'image d'analyse (raison pour laquelle il a été considéré comme optimal). Mais, dans l'agencement optimal, il n'est plus orienté selon la direction optimale.

Une nouvelle recherche d'un modèle assemblé peut donc être effectuée en modifiant les modèles de dent, par exemple en remplaçant les modèles de dent optimaux par des modèles de dent proches.

La recherche des modèles de dent à tester s'effectue de préférence au moyen d'une méthode métaheuristique, de préférence évolutionniste, de préférence par recuit simulé.

Dans le mode de réalisation préféré, le procédé met donc en œuvre une double optimisation, sur les modèles de dent et sur l'agencement des modèles de dent, le modèle assemblé étant l'agencement d'un ensemble de modèles de dent qui fournit la distance minimale avec l'image d'analyse, en considérant tous les modèles de dent possibles et tous les agencements possibles.

**A l'étape f),** optionnelle et préférée, le procédé met en œuvre plusieurs images d'analyse de l'arcade du patient, de préférence plus de 3, plus de 5, plus de 10, plus de 50, de préférence plus de 100 images d'analyse. Le modèle assemblé est ainsi plus complet. De préférence encore, le procédé met en œuvre une optimisation de manière que le modèle assemblé obtenu soit optimal au regard de l'ensemble des images d'analyse. Autrement dit, le modèle assemblé est de préférence celui qui maximise la concordance avec l'ensemble des images d'analyse.

Dans le mode de réalisation préféré, le procédé met donc en œuvre une double ou, de préférence une triple optimisation, sur les modèles de dent d'une part, sur l'agencement des modèles de dent et/ou sur une pluralité d'images d'analyse d'autre part, le modèle assemblé étant l'agencement d'un ensemble de modèles de dent qui fournit la distance minimale moyenne, sur l'ensemble des images d'analyse, en considérant tous les modèles de dent possibles et, de préférence tous les agencements possibles.

Suivant un mode de réalisation, à l'étape d) et/ou e) et/ou f), on utilise une méthode métaheuristique, de préférence évolutionniste, de préférence par recuit simulé.

Comme cela apparaît clairement à présent, l'invention permet ainsi de construire un modèle assemblé d'arcade dentaire à partir de simples images d'analyse, par exemple de photographies prises au moyen d'un téléphone mobile. Bien entendu, la précision du modèle assemblé n'atteint pas celle d'un scan. Dans certaines applications, par exemple pour effectuer un premier diagnostic de la situation dentaire du patient, une telle précision n'est cependant pas indispensable.

Le modèle assemblé peut être donc utilisé pour analyser la situation orthodontique du patient, suivant les étapes ii) à iv).

**A l'étape ii),** le modèle assemblé est envoyé à un orthodontiste et/ou à un ordinateur pourvu d'un logiciel de diagnostic.

Dans un mode de réalisation, le modèle assemblé est envoyé accompagné d'un questionnaire rempli par le patient afin d'améliorer la qualité de l'analyse à l'étape iv).

**A l'étape iii),** l'orthodontiste et/ou l'ordinateur examine le modèle assemblé. A la différence d'une image actualisée, le modèle assemblé permet une observation selon n'importe quel angle. L'analyse est avantageusement plus précise.

**A l'étape iv),** l'orthodontiste et/ou l'ordinateur informe le patient, par exemple en lui transmettant un message sur son téléphone. Ce message peut notamment informer le patient d'une situation défavorable et l'inviter à prendre un rendez-vous chez l'orthodontiste.

L'orthodontiste peut également comparer le modèle assemblé avec des modèles assemblés reçus précédemment pour le même patient. Son analyse permet avantageusement d'évaluer l'évolution de la situation. Le message peut ainsi informer le patient d'une évolution défavorable de sa situation, ce qui améliore la prévention.

Le modèle assemblé peut être également comparé avec un ou plusieurs modèles obtenus par scannage des dents ou d'un moulage des dents du patient, ou avec un modèle de référence actualisé résultant de la mise en œuvre d'un procédé décrit dans WO 2016 066651.

### Application au contrôle embarqué

Une analyse d'image selon l'invention est également utile pour guider l'acquisition d'une image d'une arcade dentaire, notamment pour l'établissement d'un diagnostic à distance.

En particulier, WO2016/066651 décrit un procédé dans lequel un modèle de référence initial est déformé de manière à obtenir un modèle de référence actualisé permettant l'acquisition d'images de référence présentant une concordance maximale avec les images « actualisées » de l'arcade acquises à l'instant actualisé.

Les images de référence sont donc des vues du modèle de référence actualisé, observé dans des conditions d'acquisition virtuelles qui sont les plus concordantes possible avec les conditions d'acquisition réelles mises en œuvre pour acquérir les images actualisées de l'arcade du patient.

La recherche de ces conditions d'acquisition virtuelles est de préférence réalisée au moyen de méthodes métaheuristiques.

Pour accélérer cette recherche, WO2016/066651 préconise d'effectuer une première évaluation grossière des conditions d'acquisition réelles. Par exemple, on exclut de la recherche des conditions qui correspondraient à une position de l'appareil d'acquisition à une distance des dents supérieure à 1 mètre.

Il existe cependant un besoin permanent pour accélérer l'exécution du procédé décrit dans WO2016/066651, et en particulier, pour rechercher, de manière plus rapide, des conditions d'acquisition virtuelles présentant une concordance maximale avec les conditions d'acquisition réelles mises en œuvre pour acquérir une image actualisée de l'arcade du patient.

Un but de l'invention est de répondre, au moins partiellement, à ce problème.

L'invention propose un procédé d'acquisition d'une image d'une arcade dentaire d'un patient, ledit procédé comportant les étapes suivantes :
a') activation d'un appareil d'acquisition d'images de manière à acquérir une image, dite « image d'analyse », de ladite arcade ;
b') analyse de l'image d'analyse au moyen d'un dispositif d'apprentissage profond, de préférence un réseau de neurones, entrainé au moyen d'une base d'apprentissage,
   de préférence suivant un procédé d'analyse détaillée selon l'invention, de manière à identifier au moins une zone de dent d'analyse représentant une dent sur ladite image d'analyse, et à déterminer au moins une valeur d'attribut de dent pour ladite zone de dent d'analyse, ou suivant un procédé d'analyse globale selon l'invention ;
c') détermination, pour l'image d'analyse, d'une valeur pour un attribut d'image, ladite valeur étant en fonction de ladite ou lesdites valeurs d'attribut de dent si un procédé d'analyse détaillée selon l'invention a été mis en œuvre à l'étape précédente ;
d') optionnellement, comparaison de ladite valeur d'attribut d'image avec une consigne ;
e') émission d'un message d'information en fonction de ladite comparaison.

Dans un mode de réalisation, à l'étape b'), on identifie toutes lesdites zones de dent d'analyse, et on détermine au moins une valeur d'attribut de dent pour chaque zone de dent d'analyse, et, à l'étape c'), on détermine la valeur pour l'attribut d'image en fonction desdites valeurs d'attribut de dent.

Dans un mode de réalisation, l'étape b') comporte les étapes suivantes :
1) de préférence avant l'étape a'), création d'une base d'apprentissage comportant plus de 1 000 images d'arcades dentaires, ou « images historiques », chaque image historique comportant une ou plusieurs zones représentant chacune une dent, ou « zones de dent historiques », à chacune desquelles, pour ledit attribut de dent, une valeur d'attribut de dent est affectée ;
2) entraînement d'au moins un dispositif d'apprentissage profond, de préférence un réseau de neurones, au moyen de la base d'apprentissage ;
3) soumission de l'image d'analyse au dispositif d'apprentissage profond de manière qu'il détermine au moins une probabilité relative à :
   - la présence, à un emplacement dans ladite image d'analyse, d'une zone représentant, au moins partiellement, une dent, ou « zone de dent d'analyse »,
   - la valeur d'attribut de la dent représentée sur ladite zone de dent d'analyse,
4) détermination, en fonction de ladite probabilité, de la présence d'une dent à une position représentée par ladite zone de dent d'analyse, et de la valeur d'attribut de ladite dent.

Dans un mode de réalisation, l'étape b') comporte les étapes suivantes :
1') création d'une base d'apprentissage comportant plus de 1 000 images d'arcades dentaires, ou « images historiques », chaque image historique comportant une valeur d'attribut pour au moins un attribut d'image, ou « valeur d'attribut d'image » ;
2') entraînement d'au moins un dispositif d'apprentissage profond, de préférence un réseau de neurones, au moyen de la base d'apprentissage ;
3') soumission de l'image d'analyse au dispositif d'apprentissage profond de manière qu'il détermine, pour ladite image d'analyse, au moins une probabilité relative à ladite valeur d'attribut d'image.

Dans un mode de réalisation, pour créer une image historique de la base d'apprentissage, un opérateur, de préférence un orthodontiste,
- identifie une ou plusieurs zones de dent « historiques » sur une image, puis affecte à chaque zone de dent historique identifiée, une valeur pour au moins un attribut de dent, et/ou
- affecte à une image une valeur pour au moins un attribut de dent.

Dans un mode de réalisation, le message d'information est émis par l'appareil d'acquisition.

Comme on le verra plus en détail dans la suite de la description, un procédé d'acquisition selon l'invention permet donc de vérifier si une image d'analyse respecte une consigne et, si elle ne respecte pas la consigne, de guider l'opérateur afin qu'il acquière une nouvelle image d'analyse. Le procédé permet donc un « contrôle embarqué », de préférence dans l'appareil d'acquisition d'images.

En particulier, pour mettre en œuvre le procédé de WO2016/066651, il peut être souhaité d'acquérir des images actualisées selon différentes direction d'acquisition, par exemple une image de face, une image de droite et une image de gauche. Ces images actualisées, acquises successivement, peuvent être classées en conséquence. La recherche des conditions d'acquisition virtuelles présentant une concordance maximale avec les conditions d'acquisition réelles en est accélérée.

En effet, la recherche peut commencer à partir de conditions d'acquisition virtuelles dans lesquelles l'appareil d'acquisition, virtuel, est en face, à gauche ou à droite du modèle de référence actualisé, selon que l'image actualisée considérée est classée comme une image de face, de gauche ou de droite, respectivement.

L'opérateur, généralement le patient, peut cependant se tromper lors de l'acquisition des images actualisées. En particulier, il peut oublier de prendre une image actualisée, par exemple la vue de face, ou inverser deux images actualisées. Typiquement, l'opérateur peut prendre une image à droite alors qu'on attend de lui une image de gauche.

Cette inversion des images actualisées peut considérablement ralentir leur traitement. Par exemple, si l'image actualisée est supposée être une image prise à gauche mais qu'elle a été par erreur prise à droite, ladite recherche des conditions d'acquisition virtuelles optimales, c'est-à-dire présentant une concordance maximale avec les conditions d'acquisition réelles, débutera d'un point de départ offrant une vue de gauche du modèle de référence, alors que les conditions d'acquisition virtuelles optimales correspondent à une vue de droite. La recherche en sera donc considérablement ralentie.

Grâce à l'invention, chaque image actualisée est une image d'analyse qui peut être analysée et contrôlée, de préférence en temps réel.

Par exemple, le procédé d'acquisition permet de déterminer que l'image actualisée a été « prise à droite » et de comparer cette valeur d'attribut d'image avec la consigne qui avait été donnée à l'opérateur de prendre l'image actualisée à gauche. La valeur d'attribut de l'image actualisée (image prise à droite) ne correspondant pas à la consigne (acquérir une image actualisée à gauche), l'appareil d'acquisition peut avertir immédiatement l'opérateur afin qu'il modifie la direction d'acquisition.

Un procédé d'acquisition est à présent décrit en détail.

**A l'étape a')**, l'opérateur active l'appareil d'acquisition d'images de manière à acquérir une image d'analyse.

Dans un mode de réalisation, l'opérateur déclenche l'appareil d'acquisition de manière à stocker l'image d'analyse, de préférence prend une photo ou une vidéo de ses dents, de préférence au moyen d'un téléphone mobile équipé d'un appareil photo.

L'étape a') peut être effectuée comme l'acquisition des images actualisées à l'étape B) décrite ci-dessus.

Dans un autre mode de réalisation, l'image d'analyse n'est pas stockée. En particulier, l'image d'analyse peut être l'image qui, en temps réel, apparaît sur l'écran du téléphone mobile de l'opérateur, généralement le patient.

Dans un premier mode de réalisation, **à l'étape b'),** on analyse l'image d'analyse suivant un procédé d'analyse détaillée selon l'invention. Cette analyse conduit de préférence à l'affectation d'une valeur d'attribut de dent à chaque zone de dent d'analyse identifiée, par exemple à affecter un numéro de dent à chacune des zones de dent d'analyse.

**A l'étape c'),** on détermine une valeur d'attribut de l'image d'analyse en fonction des valeurs d'attribut de dent. La valeur d'attribut de l'image d'analyse peut être relative à son orientation générale et peut par exemple prendre une des trois valeurs suivantes : « photo de droite », « photo de gauche » et « photo de face ». La valeur d'attribut de l'image d'analyse peut également être la liste des numéros des dents représentées, par exemple, « 16, 17 et 18 ». La valeur d'attribut de l'image d'analyse peut encore être, par exemple, la « présence » ou « l'absence » d'un appareil dentaire, de préférence orthodontique, ou l'état d'ouverture de la bouche (« bouche ouverte », « bouche fermée »).

Dans un autre mode de réalisation, un procédé d'analyse globale selon l'invention est mis en œuvre à l'étape b'). Avantageusement, un tel procédé permet d'obtenir directement une valeur pour un attribut d'image, sans avoir à déterminer de valeurs pour un attribut de dent. Il est donc avantageusement plus rapide. L'information résultant d'une analyse globale peut être cependant moins précise que celle résultant d'une analyse détaillée.

Les étapes a') à c') permettent ainsi de caractériser l'image d'analyse.

La caractérisation de l'image d'analyse permet de guider l'opérateur si l'image d'analyse ne correspond pas à l'image attendue, par exemple parce que sa qualité est insuffisante ou parce qu'elle ne représente pas les dents souhaitées.

**A l'étape d'),** on compare la valeur d'attribut d'image de l'image d'analyse avec une consigne.

Par exemple, si la consigne était d'acquérir une image de droite et que la valeur d'attribut d'image est « prise à gauche », la comparaison conduit à la conclusion que l'image acquise est « insatisfaisante ».

**A l'étape e'),** un message est envoyé à l'opérateur, de préférence par l'appareil d'acquisition.

De préférence, le message d'information est relatif à la qualité de l'image acquise et/ou à la position de l'appareil d'acquisition par rapport à ladite arcade et/ou au réglage de l'appareil d'acquisition et/ou à l'ouverture de la bouche et/ou au port d'un appareil dentaire, de préférence orthodontique.

Par exemple, si l'image acquise est « insatisfaisante », l'appareil d'acquisition peut émettre une lumière, par exemple rouge, et/ou sonner, et/ou générer un message vocal, et/ou vibrer, et/ou afficher un message sur son écran.

Par exemple, si l'image doit être acquise alors que le patient porte son appareil dentaire et que ce n'est pas le cas, l'appareil d'acquisition peut émettre le message « portez votre appareil pour cette image ».

Par exemple, si l'image a été acquise alors que le patient n'ouvre pas suffisamment la bouche ou a la bouche fermée, l'appareil d'acquisition peut émettre le message « ouvrez plus votre bouche pour cette image ».

Dans un mode de réalisation, les étapes b') à c') ne sont mises en œuvre que si l'opérateur enregistre l'image d'analyse, c'est-à-dire qu'il appuie sur le déclencheur. Le message invite alors l'opérateur à acquérir une nouvelle image d'analyse. Optionnellement, l'appareil d'acquisition efface l'image d'analyse insatisfaisante.

Dans un mode de réalisation, les étapes b') à c') sont mises en œuvre en permanence quand l'appareil d'acquisition est en marche et l'image d'analyse est une image qui apparaît sur un écran de l'appareil d'acquisition. L'appareil d'acquisition peut ainsi, par exemple, émettre une lumière rouge tant que l'image d'analyse est insatisfaisante, et émettre une lumière verte quand l'image d'analyse est satisfaisante. Avantageusement, l'appareil d'acquisition ne stocke alors que des images d'analyse qui sont satisfaisantes.

Comme cela apparaît clairement à présent, l'invention permet donc un contrôle embarqué lors de l'acquisition d'images d'analyse. Appliquées à des images actualisées du procédé de WO2016/066651, les étapes a') à e') permettent de s'assurer que ces images sont bien conformes au besoin, et donc d'accélérer considérablement l'exécution de ce procédé.

Les étapes d') et e') sont optionnelles. Dans un mode de réalisation, l'image d'analyse est seulement associée à son descriptif, qui précise sa valeur d'attribut d'image. Ce descriptif permet également d'accélérer considérablement l'exécution du procédé de WO2016/066651 puisque, lorsque l'image d'analyse est utilisée comme image actualisée de ce procédé, il permet de déterminer approximativement les conditions d'acquisition réelle de cette image, en éliminant le risque d'une erreur grossière, par exemple du fait d'une inversion entre deux images.

Les étapes d') et e') sont cependant préférées. Elles permettent par exemple d'éviter que l'opérateur oublie une image de gauche, ou prenne deux images de droite redondantes.

### Application au contrôle d'une gouttière orthodontique

Classiquement, au début d'un traitement orthodontique, l'orthodontiste détermine le positionnement des dents qu'il souhaite obtenir à un instant du traitement, dit "set-up ". Le set-up peut être défini au moyen d'une empreinte ou à partir d'un scan tridimensionnel des dents du patient. L'orthodontiste fait fabriquer ou fabrique alors, en conséquence, un appareil orthodontique adapté à ce traitement.

L'appareil orthodontique peut être une gouttière orthodontique (« *aligner »* en anglais). Une gouttière se présente classiquement sous la forme d'un appareil monobloc amovible, classiquement en un matériau polymère transparent, qui comporte une goulotte conformée pour que plusieurs dents d'une arcade, généralement toutes les dents d'une arcade, puissent y être logées.

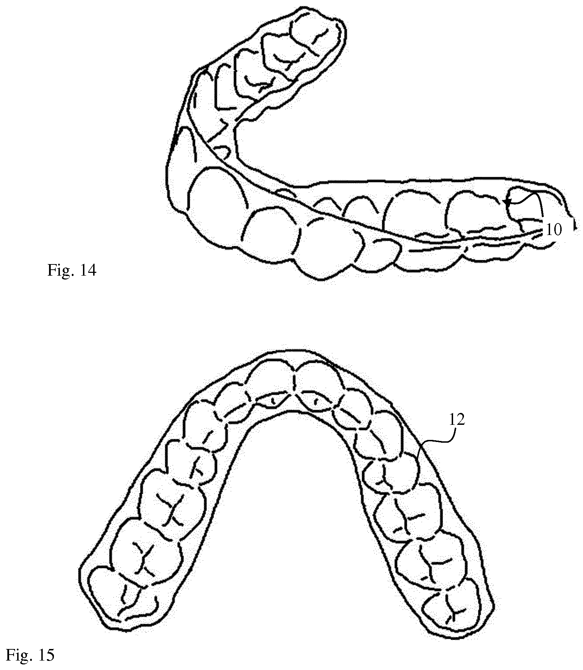

La forme de la goulotte est adaptée pour maintenir en position la gouttière sur les dents, tout en exerçant une action de correction du positionnement de certaines dents (figures 14 et 15).

On détermine classiquement, au début du traitement, les formes que doivent prendre les différentes gouttières à différents moments du traitement, puis on fait fabriquer l'ensemble des gouttières correspondantes. A des instants prédéterminés, le patient change de gouttière.

Le traitement au moyen de gouttières est avantageusement peu contraignant pour le patient. En particulier, le nombre de rendez-vous chez l'orthodontiste est limité. En outre, la douleur est plus faible qu'avec un arc orthodontique métallique attaché aux dents.

Le marché des gouttières orthodontiques est donc en croissance.

A intervalles réguliers, le patient se déplace chez l'orthodontiste pour un contrôle visuel, notamment pour vérifier si le déplacement des dents est conforme aux attentes et si la gouttière portée par le patient est toujours adaptée au traitement.

Si l'orthodontiste diagnostique une inadaptation au traitement, il effectue une nouvelle empreinte des dents, ou, de manière équivalente, un nouveau scan tridimensionnel des dents, puis commande une nouvelle série de gouttières configurées en conséquence. On considère qu'en moyenne, le nombre de gouttières finalement fabriquées est d'environ 45, au lieu des 20 gouttières classiquement prévues au début du traitement.

La nécessité de devoir se déplacer chez l'orthodontiste est une contrainte pour le patient. La confiance du patient en son orthodontiste peut être également entamée. L'inadaptation peut être inesthétique. Enfin, il en résulte un coût supplémentaire.

Le nombre de visites de contrôle chez l'orthodontiste doit donc être limité.

Il existe un besoin pour des solutions répondant à ces problèmes.

Un but de l'invention est de répondre, au moins partiellement, à ce besoin.

L'invention fournit un procédé d'évaluation de la forme d'une gouttière orthodontique, ledit procédé comportant les étapes suivantes :
a") acquisition d'au moins une image représentant au moins partiellement la gouttière dans une position de service dans laquelle elle est portée par un patient, dite « image d'analyse » ;
b") analyse de l'image d'analyse au moyen d'un dispositif d'apprentissage profond, de préférence un réseau de neurones, entrainé au moyen d'une base d'apprentissage, de manière à déterminer une valeur
   pour au moins un attribut de dent d'une « zone de dent d'analyse » de l'image d'analyse, l'attribut de dent étant relatif à un écartement entre la dent représentée par la zone de dent d'analyse, et la gouttière représentée sur l'image d'analyse, et/ou pour un attribut d'image de l'image d'analyse, l'attribut d'image étant relatif à un écartement entre au moins une dent représentée sur l'image d'analyse, et la gouttière représentée sur ladite image d'analyse ;
c") de préférence, évaluation de l'adéquation de la gouttière en fonction de la valeur dudit attribut de dent ou d'image ;
d") de préférence, émission d'un message d'information en fonction de ladite évaluation.

Comme on le verra plus en détail dans la suite de la description, un procédé d'évaluation selon l'invention facilite considérablement l'évaluation de la bonne adéquation de la gouttière au traitement, tout en rendant cette évaluation particulièrement fiable. En particulier, le procédé peut être mis en œuvre à partir de simples photographies ou films, pris sans précaution particulière, par exemple par le patient. Le nombre de rendez-vous chez l'orthodontiste peut donc être limité.

De préférence, à l'étape b''), on identifie toutes lesdites zones de dent d'analyse, et on détermine la valeur dudit attribut de dent pour chaque zone de dent d'analyse, et, à l'étape c"), on détermine l'adéquation de la gouttière en fonction desdites valeurs d'attribut de dent.

De préférence, ledit attribut de dent est choisi dans le groupe formé par un écartement maximal le long de bord libre de la dent, un écartement moyen le long de bord libre de la dent, et ledit attribut d'image est choisi dans le groupe formé par un écartement maximal le long de l'ensemble des dents représentées, un écartement moyen le long des bords libres de l'ensemble des dents représentées, une acceptabilité globale de l'écartement des dents représentées.

L'attribut de dent relatif à un écartement peut être en particulier l'existence d'un écartement, cet attribut pouvant prendre les valeurs d'attribut de dent « oui » ou « non » ; ou une valeur mesurant l'ampleur de l'écartement, par exemple un écartement maximal constaté ou une évaluation relative à une échelle.

A l'étape b''), on met de préférence en œuvre un procédé d'analyse détaillée selon l'invention, un attribut de dent de chaque zone de dent historique de chaque image historique de la base d'apprentissage étant relatif à un écartement entre la dent représentée par la zone de dent historique, et une gouttière portée par ladite dent et représentée sur ladite image historique.

De préférence, l'étape b") comporte les étapes suivantes :
b"1) de préférence avant l'étape a"), création d'une base d'apprentissage comportant plus de 1 000, de préférence plus de 5 000, de préférence plus de 10 000 images d'arcades dentaires, ou « images historiques », chaque image historique représentant une gouttière portée par un patient « historique » et comportant une ou plusieurs zones représentant chacune une dent, ou « zones de dent historiques », à chacune desquelles, pour au moins un attribut de dent relatif à un écartement entre la dent représentée par la zone de dent historique considérée, et la gouttière représentée, une valeur d'attribut de dent est affectée ;
b"2) apprentissage d'un dispositif d'apprentissage profond, de préférence un réseau de neurones, au moyen de la base d'apprentissage ;
b"3) soumission de l'image d'analyse au dispositif d'apprentissage profond de manière que le dispositif d'apprentissage profond détermine au moins une probabilité relative à
   - la présence, à un emplacement de ladite image d'analyse, d'une zone de dent d'analyse, et
   - la valeur d'attribut de dent de la dent représentée sur ladite zone de dent d'analyse ;
b"4) détermination, en fonction de ladite probabilité, de la présence d'un écartement entre la gouttière et la dent représentée par ladite zone de dent d'analyse, et/ou d'une amplitude dudit écartement.

Les étapes b"1) à b"4) peuvent comporter une ou plusieurs des caractéristiques, éventuellement optionnelles, des étapes 1) à 4) décrites ci-dessus, respectivement.

Dans un mode de réalisation, à l'étape b''), on met en œuvre un procédé d'analyse globale selon l'invention, un attribut d'image de chaque image historique de la base d'apprentissage étant relatif à un écartement entre au moins une dent représentée sur l'image historique, et une gouttière portée par ladite dent et représentée sur ladite image historique.

De préférence, l'étape b") comporte les étapes suivantes :
b"1') création d'une base d'apprentissage comportant plus de 1 000 images d'arcades dentaires, ou « images historiques », chaque image historique comportant une valeur d'attribut pour au moins un attribut d'image, ou « valeur d'attribut d'image », relatif à un écartement entre au moins une dent représentée sur l'image d'analyse, et la gouttière représentée sur ladite image d'analyse ;
b"2') entraînement d'au moins un dispositif d'apprentissage profond, de préférence un réseau de neurones, au moyen de la base d'apprentissage ;
b"3') soumission de l'image d'analyse au dispositif d'apprentissage profond de manière qu'il détermine, pour ladite image d'analyse, au moins une probabilité relative à ladite valeur d'attribut d'image, et détermination, en fonction de ladite probabilité, de la présence d'un écartement entre la gouttière et la ou les dents représentées sur l'image d'analyse, et/ou d'une amplitude dudit écartement.

Les étapes b''1') à b''3') peuvent comporter une ou plusieurs des caractéristiques, éventuellement optionnelles, des étapes 1') à 3') décrites ci-dessus, respectivement.

Le procédé est à présent décrit lorsqu'une analyse détaillée est mise en œuvre à l'étape b").

**Préalablement à l'étape a")**, la base d'apprentissage doit être enrichie, de préférence suivant un procédé d'enrichissement selon l'invention, afin de contenir des images historiques dont le descriptif précise, pour chacune des zones de dent historiques, une valeur pour l'attribut de dent relatif à l'écartement.

Cette information peut être saisie manuellement. Par exemple, on peut présenter à un opérateur, de préférence à un orthodontiste, une image représentant une ou plusieurs zones de dent dites « historiques », et lui demander d'identifier ces zones de dent historiques et d'indiquer, pour chaque zone de dent historique, s'il y a un écartement ou pas et/ou d'évaluer l'amplitude de cet écartement.

Une image historique peut être une photo représentant une gouttière portée par un patient historique. Alternativement, une image historique peut être le résultat d'un traitement d'une image représentant une arcade dentaire nue (c'est-à-dire sans gouttière) et d'une image représentant la même arcade portant la gouttière. L'image représentant l'arcade nue peut en particulier être une vue d'un modèle de l'arcade déformé pour obtenir une concordance maximale avec l'image représentant l'arcade portant la gouttière. Un tel traitement peut être en particulier utile pour faire mieux apparaître le contour des dents et de gouttière lorsque les dents sont peu visibles à travers la gouttière.

**A l'étape a"),** l'acquisition de l'image d'analyse peut être effectuée comme l'acquisition des images actualisées à l'étape B) décrite ci-dessus.

De préférence, au moins un rappel informant le patient de la nécessité de créer une image d'analyse est adressé au patient. Ce rappel peut être sous forme papier ou, de préférence, sous forme électronique, par exemple sous la forme d'un courriel, d'une alerte automatique d'un applicatif spécialisé mobile ou d'un SMS. Un tel rappel peut être envoyé par le cabinet ou le laboratoire d'orthodontie ou par le dentiste ou par l'applicatif spécialisé mobile du patient, par exemple.

L'étape a") est réalisée au moment où l'évaluation de la forme d'une gouttière est souhaitée, par exemple plus de 1 semaine après le début du traitement avec la gouttière.

L'image d'analyse est une image représentant la gouttière portée par les dents du patient.

**A l'étape b"),** on analyse l'image d'analyse suivant un procédé d'analyse détaillée selon l'invention.

Le dispositif d'apprentissage profond a été entrainé au moyen d'une base d'apprentissage contenant des images historiques dont le descriptif précise, pour au moins une, de préférence chaque zone de dent historique, une valeur pour un attribut de dent relatif à un écartement entre la dent représentée par la zone de dent historique et la gouttière portée par ladite dent et représentée sur ladite image historique.

La valeur pour cet attribut de dent fournit donc une information relative à la forme de la gouttière relativement à la forme des dents du patient.

La valeur pour cet attribut de dent peut être une mesure de l'écartement, par exemple une mesure de l'écartement maximal, ou de l'écartement moyen pour la dent représentée par la zone de dent historique.

Le dispositif d'apprentissage profond est donc capable d'analyser l'image d'analyse pour déterminer, de préférence pour chacune des « zones de dent d'analyse », l'existence, voire l'importance d'un écartement de la gouttière de la dent représentée sur la zone de dent d'analyse.

**A l'étape c"),** on évalue, en fonction des résultats de l'étape précédente, l'adéquation de la gouttière. Par exemple, on recherche si l'écartement de la gouttière avec au moins une dent dépasse un seuil d'acceptabilité et, dans ce cas, on décide du remplacement de la gouttière par une gouttière mieux adaptée.

L'adéquation de la gouttière peut être évaluée dans le cadre d'un traitement orthodontique (écartement compatible ou non avec le traitement orthodontique), mais aussi dans le cadre d'un traitement non thérapeutique, notamment esthétique. Des gouttières peuvent être en effet utilisées pour déplacer des dents à des fins purement esthétiques, sans que ce déplacement ne modifie l'état de santé du patient. L'adéquation de la gouttière peut être également évaluée dans le cadre d'un programme de recherche sur l'efficacité de la gouttière, par exemple pour évaluer un nouveau matériau pour la gouttière, sur un être humain ou sur un autre animal.

**A l'étape d"),** une information relative à l'évaluation effectuée à l'étape précédente est émise, en particulier à destination du patient et/ou de l'orthodontiste.

L'orthodontiste peut alors utiliser cette information, éventuellement en combinaison avec des informations complémentaires, par exemple l'âge du patient ou la durée pendant laquelle la gouttière a été portée, pour établir un diagnostic et, le cas échéant, décider d'un traitement adapté.

Dans un mode de réalisation, le procédé comporte, à l'étape b''), une analyse globale selon l'invention. Les autres étapes ne sont pas modifiées.

L'analyse de l'image d'analyse et des images historiques est alors effectuée globalement, sans identification de la situation individuelle de chacune des dents représentées et l'attribut d'image est relatif à l'image dans sa globalité.

Par exemple, l'attribut d'image relatif à l'écartement peut être relatif à l'acceptabilité d'une situation dentaire, du fait d'un ou plusieurs écartements, ou relatif à l'ampleur globale du ou des écartements des dents. Par exemple, la valeur de l'attribut d'image peut être « globalement acceptable » ou « globalement inacceptable ». La valeur de l'attribut d'image peut être également, par exemple, une mesure de l'écartement, par exemple une mesure de l'écartement maximal, ou de l'écartement moyen entre les dents représentées sur l'image d'analyse et la gouttière.

Comme cela apparaît clairement à présent, un procédé selon l'invention permet, à partir de simples photos ou d'un simple film, de déterminer si la gouttière est anormalement décollée, voire, si une analyse détaillée a été effectuée à l'étape b"), de déterminer les régions dans lesquelles la gouttière s'est écartée des dents et d'évaluer l'ampleur de cet écartement.

L'invention concerne aussi un procédé d'adaptation d'un traitement orthodontique, procédé dans lequel on met en œuvre un procédé d'évaluation de la forme d'une gouttière orthodontique selon l'invention, puis, en fonction du résultat de ladite évaluation, on fabrique une nouvelle gouttière et/ou on conseille le patient, par exemple pour qu'il améliore les conditions d'utilisation de sa gouttière orthodontique, en particulier le positionnement et/ou les plages horaires de port et/ou l'entretien de sa gouttière orthodontique, afin d'optimiser le traitement.

L'utilisation de gouttières n'est pas limitée à des traitements thérapeutiques. En particulier, un procédé d'évaluation peut être mis en œuvre pour évaluer une gouttière exclusivement utilisée à des fins esthétiques.

Le procédé peut être également utilisé pour évaluer d'autre pièces ou appareils dentaires, notamment orthodontiques.

### Programme d'ordinateur

L'invention concerne également :
- un programme d'ordinateur, et en particulier un applicatif spécialisé pour téléphone mobile, comprenant des instructions de code de programme pour l'exécution d'une ou plusieurs étapes d'un procédé quelconque selon l'invention, lorsque ledit programme est exécuté par un ordinateur,
- un support informatique sur lequel est enregistré un tel programme, par exemple une mémoire ou un CD-ROM.

Bien entendu, l'invention n'est pas limitée aux modes de réalisation décrits ci-dessus et représentés.

En particulier, le patient n'est pas limité à un être humain. Un procédé selon l'invention peut être utilisé pour un autre animal.

Le patient peut être vivant ou mort. Il est de préférence vivant.

Les procédés selon l'invention peuvent être mis en œuvre dans le cadre d'un traitement orthodontique, mais aussi hors de tout traitement orthodontique, et même hors de tout traitement thérapeutique.

## Revendications

1. Procédé d'analyse d'une image d'une arcade dentaire d'un patient, dite « image d'analyse », comportant l'acquisition avec un téléphone mobile et par le patient de l'image d'analyse, l'image d'analyse étant en couleurs et choisie parmi une photographie et une image extraite d'un film, le téléphone mobile étant écarté de l'arcade dentaire de plus de 5 cm lors de l'acquisition, ledit procédé d'analyse comportant les étapes suivantes :
1') création d'une base d'apprentissage comportant plus de 1 000 images d'arcades dentaires, ou « images historiques », chaque image historique comportant une valeur d'attribut pour un attribut d'image, ou « valeur d'attribut d'image » ;
2') entraînement d'au moins un réseau de neurones, au moyen de la base d'apprentissage, en présentant, en entrée du réseau de neurones, les images historiques, afin que le réseau de neurones apprenne progressivement à reconnaître sur une image, des motifs, et à les associer à des valeurs d'attribut d'image ;
3') soumission de l'image d'analyse au réseau de neurones de manière qu'il détermine, pour ladite image d'analyse, au moins une probabilité relative à ladite valeur d'attribut d'image, et détermination, en fonction de ladite probabilité, d'une valeur pour ledit attribut d'image pour l'image d'analyse ;
ledit attribut d'image étant relatif à
- une position et/ou une orientation et/ou une calibration de l'appareil d'acquisition utilisé pour acquérir ladite image d'analyse, ou
- une qualité de l'image d'analyse, ou
- la luminosité, au contraste ou à la netteté de l'image d'analyse,

2. Procédé selon la revendication immédiatement précédente, dans lequel à l'étape 1'), pour créer une image historique de la base d'apprentissage, un opérateur, de préférence un orthodontiste, affecte à une image une valeur pour au moins un attribut de dent.

3. Procédé selon l'une quelconque des deux revendications immédiatement précédentes, dans lequel, à l'étape 1), on crée une base d'apprentissage comportant plus de 10 000 images historiques.

4. Procédé selon l'une quelconque des revendications précédentes, comportant les étapes suivantes :
a') activation d'un appareil d'acquisition d'images de manière à acquérir l'image d'analyse,
b') analyse de l'image d'analyse au moyen d'un réseau de neurones, entrainé au moyen d'une base d'apprentissage, selon un procédé selon l'une quelconque des revendications précédentes.

5. Procédé selon la revendication 4, comportant l'étape d') :
comparaison de ladite valeur d'attribut d'images avec une consigne.

6. Procédé selon la revendication immédiatement précédente, comportant l'étape e') :
émission d'un message d'information en fonction de ladite comparaison, de préférence par le téléphone mobile.

7. Procédé selon l'une quelconque des revendications précédentes, l'image d'analyse représentant plusieurs dents, de préférence plus de 2, plus de 3, plus de 4 ou plus de 5 dents du patient.

8. Procédé selon l'une quelconque des revendications précédentes, dans lequel le déclenchement de l'acquisition est automatique, c'est-à-dire sans action d'un opérateur, lorsque l'appareil d'acquisition d'images détermine qu'il observe une arcade dentaire et/ou un écarteur et que les conditions d'observations sont satisfaisantes.

9. Procédé selon l'une quelconque des revendications précédentes, dans lequel le réseau de neurones est un R-CNN, un Single Shot MultiBox Detector, ou un Faster Region-based Convolutional Network.
